# EUROPEAN PATENT APPLICATION

(11) **EP 2 913 328 A1**
(43) Date of publication of application: **02.09.2015**
(21) Application number: 13849866.2
(22) Date of filing: 24.10.2013
(51) Int. Cl.: C07D 307/10, C07D 307/12, C10L 1/02, C10L 1/18

(54) **CATALYST AND CATALYTIC PROCESS FOR THE ETHERIFICATION/REDUCTION OF FURFURYL DERIVATIVES TO TETRAHYDROFURFURYL ETHERS**

(30) Priority: 25.10.2012 ES 201231645
(71) Applicant: Consejo Superior De Investigaciones Científicas (CSIC), 28006 Madrid (ES); Universitat Politècnica de València, 46022 Valencia (ES)
(72) Inventor: CORMA CANÓS, Avelino, 46022 Valencia (ES); DÓMINE, Marcelo Eduardo, 46022 Valencia (ES); VALENCIA VALENCIA, Susana, 46022 Valencia (ES)
(74) Representative: Cueto, Sénida
(86) International application number: PCT/ES2013/070739
(87) International publication number: WO 2014/064318

(57) **Abstract**

The invention relates to a method for producing tetrahydrofurfuryl ethers, characterised in that it involves carrying out consecutive etherification/reduction reactions based on a compound containing at least one furan ring, in the presence of at least one alcohol and at least one catalyst, optionally in the presence of H₂. The catalytic process can be carried out in a cascade reaction ("one-pot"), operating under soft reaction conditions and without a solvent.

## Description

### Field of the technique

Etherification. Hydrogenation. Heterogeneous Catalysis. Petrochemistry.

### Background

The use of biomass and its derivatives as raw materials for the chemical industry acquires more general interest every day [P. Gallezot, ChemSusChem, 1, 586, 2008]. Biomass is, together with CO₂, one of the primary and renewable coal sources, and the revalorization of its derivatives becomes a sustainable alternative to fossil hydrocarbons. In this context, and after an initial treatment (for example, through fermentation, hydrolysis, thermal and/or catalytic processes) of lignocellulosic type biomass, derivative compounds, such as sugars glycerol and its derivatives, furfural, 5-hydroxymethyl-furfural and levulinic acid, among many others, can be obtained, with relative ease, [M. Stocker, Angew. Chem. Int. Ed., 47, 2, 2008; G. W. Huber et al., Chem. Rev., 106, 4044, 2006; G. W. Huber et al., Angew. Chem. Int. Ed., 46, 7184, 2007; J. N. Chheda et al., Angew. Chem. Int. Ed., 46, 7164, 2007; A. Corma et al., Chem. Rev., 107, 2441, 2007]. These biomolecules can be used as new starting compounds ("Platform Chemicals") for the synthesis of chemicals and components and additives for liquid fuels in automotive industry [G. W. Huber et al., Angew. Chem. Int. Ed., 46, 7184, 2007; J. N. Chheda et al., Angew. Chem. Int. Ed., 46, 7164, 2007; A. Corma et al., Chem. Rev., 107, 2441, 2007].

Direct etherification reaction of alcohols and polyalcohols derived from biomass with olefins to obtain compounds that can be used as additives in liquid fuels [F. Jérôme et al., ChemSusChem, 1, 586, 2008] and chemicals [M. Pagliaro et al., Angew. Chem. Int. Ed., 46, 4434, 2007] is one of the most representative industrial applications. Thus, the use of bio-alcohols in the etherification of iso-butene and iso-amylene to produce mono-alkyl ethers (for example, MTBE, ETBE, TAME) commonly used so far as anti-knock additives and octane fuel improvers has been extensively studied using acid catalysts [N. V. Vlasenko et al., Appl. Catal. A: Gral., 362(1-2), 82, 2009; and J. Mol. Catal. A: Chem., 253(1-2), 192, 2006; P. K. Pääkkönen et al., Appl. Catal. A: Gral., 245, 289, 2003]. Recently, direct etherification of glycerol (and ethyleneglycol) with C4-C8 olefins for the production of C4-C8-mono-and di- alkyl-ethers with interesting applications as non-ionic surfactants was reported, using acid resins as catalysts [R. S. Karinen et al., Appl. Catal. A: Gral., 306, 128, 2006], zeolites (H-BEA and H-Y) or sulfonated mesoporous silica [A. M. Ruppert et al., J. Catal., 268, 251, 2009; J. A. Melero et al., Appl. Catal. A: Gral., 346, 44, 2008].

In recent years, the use of sugars and its furan derivatives, such as furfural (FAL) and 5-hydroxymethyl-furfural (HMF) as versatile and alternative raw materials for obtaining a variety of chemical products and derivatives in different industrial processes has generated growing interest in the scientific community [G. W. Huber et al., Angew. Chem. Int. Ed., 46, 7184, 2007; J. N. Chheda et al., Angew. Chem. Int. Ed., 46, 7164, 2007; A. Corma et al., Chem. Rev., 107, 2441, 2007; C. Moreau et al., Topics in Catal., 27(1), 11, 2004]. Thus, for example, mixtures of long chain oxygenated hydrocarbons (C6-C15) and saturated oxygenated heterocycles soluble in water, which can be used as additives for liquid fuels and alkane precursors, can be produced through consecutive aldol condensation / hydrogenation reactions, from an aqueous solution of HMF and acetone in the presence of bi-functional metal oxide type catalysts, being the HMF first generated via direct dehydration of fructose catalyzed by a mineral acid, [J. N. Chheda et al., Catal. Today, 123, 5915, 2007].

Recently, furfuril- and tetra-hydro-furfuryl ethers were prepared from the corresponding 5-alcoxi-methyl furfural by consecutive decarbonylation and decarbonylation / hydrogenación reactions, respectively, and their possible use as components and additives for jet fuels has been claimed [G. J. M. Gruter et al. (Furanix Technologies B.V.), EP 2128227 A1, 2009; G. J. M. Gruter et al. (Avantium International B.V.), EP 1834950 A1, 2007]. In this case, the initial etherification reaction of HMF has been carried out with primary alcohols (C1-C4) in the presence of a mineral acid as catalyst [G. J. M. Gruter et al. (Avantium International B.V.), EP 1834950 A1, 2007]. It is also possible to synthesize these mono-ethers furan derivatives starting from the corresponding furfuryl alcohol, [G. J. M. Gruter et al. (Furanix Technologies B.V.), EP 2128227 A1, 2009; G. J. M. Gruter et al. (Avantium International B.V.), EP 1834950 A1, 2007]. Finally, it has also been reported the direct etherification of HMF with olefins catalyzed by acid (for example, mineral acids, bentonites, Sc (III) and Sm (III)) triflates to produce the corresponding alkyl mono-ethers with interesting applications as anti-knock additive in liquid fuels [G. J. M. Gruter (Furanix Technologies B.V.), WO 030505 A2, 2009; and G. J. M. Gruter (Furanix Technologies B.V.), WO 030504 A2, 2009].

Finally, it has been reported the use of combinations of solid catalysts capable of producing the consecutive hydrogenation reactions of furfuryl aldehyde to derivatives of tetrahydro furfuryl alcohol type, in a cascade type process, and their subsequent etherification in the presence of alcohols to obtain the corresponding tetrahydro-furfuryl ethers, by a heterogeneous catalytic process [G. J. M. Gruter et al. (Furanix Technologies B.V.), WO 030509 A2, 2009; and G. J. M. Gruter et al. (Furanix Technologies B.V.), WO 030510 A2, 2009]. However, in these cases the use of a hydrogenation catalyst of the Ni/SiO₂, type, highly dangerous and with highly risky manipulation, is also necessary, besides working with H₂ pressures above 10 bar, and in the presence of high nonreactive solvent volumes. Moreover, working under these reaction conditions mixtures of non-hydrogenated etherified furfuryl derivatives and hydrogenated non-etherified furfuryl derivatives with a varied and little selective product distribution and total yield (sum of all derivatives obtained) of less than 72%, are obtained as end products of the process [G. J. M. Gruter et al. (Furanix Technologies B.V.), WO 030510 A2, 2009].

From the foregoing, the need to develop a highly selective catalytic process is evident, that allows to obtain tetrahydro-furfuryl ethers, or mixtures of furfuryl ethers and tetrahydro-furfuryl ethers in a simple and efficient manner, from furfural or furfuryl derivatives and alcohols, using a catalyst or a combination of solid catalysts for carrying out etherification / hydrogenation consecutively in a cascade reaction ("one pot"), i.e., in the same reactor, working under mild reaction conditions (low temperatures and low H₂ pressures) and in the absence of solvent. In this manner, the reduction of reaction steps, not using large solvent amounts, and the use of mild reactions conditions will allow to reduce the risks involved in the hydrogenation reactions, as well as increasing the energy saving, considerably increasing the benefit /cost ratio in the global process. In this regard, the design of new cascade catalytic processes that avoid using hydrogenation catalysts such as Ni/SiO₂ in high amounts is critical. Furthermore, if the process is preferably performed in a cascade reaction ("one-pot") and with a single multi-functional catalyst, competitive additional advantages will become significantly higher.

### Description of the invention

The present invention refers to a process for obtaining tetrahydro-frufuril ethers comprising performing an etherification / reduction consecutively in a cascade reaction, from a compound containing one or more furan rings, preferably a mono-furfuryl derivative, and more preferably 5-hydroxymethyl furfural and furfural, in absence of solvent and in the presence of at least:
- one or more alcohols,
- one or more catalysts, and
wherein the etherification reaction is preferably carried out in absence of hydrogen and the reduction reaction is preferably carried out under the presence of hydrogen.

According to a particular embodiment the etherification reaction can be carried out in the presence of hydrogen.

According to one embodiment of the present invention, compounds of tetrahydro-furfuryl ether type are preferably derivatives with a-tetrahydrofuran ring containing at least one or more ether type oxygenated substituents (-H₂C-OR), R being a linear or cyclic aliphatic hydrocarbon substituent, with none, one or more chain branches, and comprising a hydrocarbon chain of between 1 and 24 carbon atoms; an aromatic substituent, with none, one or more substituents in the ring and comprising a hydrocarbon chain with 6 to 18 carbons, and sometimes containing the tetrahydrofuran ring one or more oxygenated substituents of alcohol type (H₂C-OH) alkoxide (H₂C-OR, R being an alkyl or aryl group), carboxyl (-COOH), carboxylate (COOR, where R is an alkyl or aryl group); one or more substituents of aliphatic or aromatic oxygenated heterocycle type, substituted or unsubstituted, and comprising a hydrocarbon chain from 4 to 12 carbons, one or more linear or cyclic aliphatic hydrocarbon substituents, with none, one or more chain branches, and that comprise a hydrocarbon chain of between 1 to 24 carbon atoms. Examples of these tetrahydro-furfuryl ethers are for example 2-iso-propoxymethyl-tetrahydro-furfuryl ether, 2-sec-butoxymethyl-tetrahydro-furfuryl ether, 2-(2-hexoxy)-methyl-tetrahydro-furfuryl ether, 2-(2-octoxy)-methyl-tetrahydro-furfuryl ether, 2-iso-propoxymethyl-5-hydroxymethyl-tetrahydro-furfuryl ether, 2-sec-butoxymethyl-5-hydroxymethyl-tetrahydro-furfuryl ether, 2-(2-hexoxy)-methyl-5-hydroxymethyl-tetrahydro-furfuryl ether, 2-(2-octoxy)-methyl-5-hydroxymethyl-tetrahydro-furfuryl ether, 2-iso-propoxymethyl-5-methoxymethyl-tetrahydro-furfuryl ether, 2-sec-butoxymethyl-5-methoxymethyl-tetrahydro-furfuryl ether, 2-(2-hexoxy) methyl-5-methoxymethyl-tetrahydro-furfuryl ether, 2-(2-octoxy)-methyl-5-methoxymethyl-tetrahydro-furfuryl ether, 2-iso-propoxymethyl-5-ethoxymethyl-tetrahydro-furfuryl ether, 2-sec-butoxymethyl-5-ethoxymethyl-tetrahydro-furfuryl ether, 2-(2-hexoxy)-methyl-5-ethoxymethyl-tetrahydro-furfuryl ether, 2-(2-octoxy)-methyl-5-ethoxymethyl-tetrahydro-furfuryl ether, among others, without these being limiting examples.

According to one embodiment of the invention, compounds of the tetrahydro-furfuryl ether type containing at least one tetrahydrofuran ring can have the general formula: 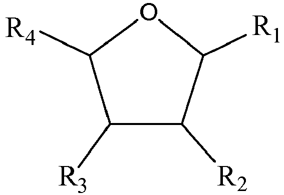 wherein R₁ is a -H₂C-OR group, R being a linear or cyclic hydrocarbon substituent, with none, one or more chain branches, and that comprises a hydrocarbon chain of between 1 y 24 carbon atomss; an aromatic substituent, with none, one or more substituents in the ring, and which comprises a hydrocarbon chain of between 6 and 18 carbon atoms. R₂, R₃ and R₄ are substituents identical or different from each other, and are indistinctly selected from among hydrogen, oxygenated substituent of alcohol type (H₂C-OH), alkoxide (H₂C-OR, R being alkyl or aryl group), carboxyl (-COOH), carboxylate (COOR, where R is an alkyl or aryl group), aliphatic or aromatic oxygenated heterocycle with 4 to 12 C atoms, substituted or unsubstituted, alkyl having 1 to 24 carbon atoms, linear or branched, substituted or unsubstituted; cyclic alkyl having 4 to 24 carbon atoms, substituted or unsubstituted; or aryl having 6 to 18 carbon atoms, substituted or unsubstituted.

According to one embodiment of the present invention, compounds of the tetrahydro-furfuryl ether type containing at least one ring tetrahydrofuran may possess the general formula: 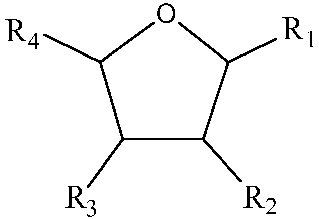 wherein R₁ is a -H₂C-OR group, R being a linear or cyclic aliphatic hydrocarbon substituent, with none, one or more chain branches, and comprising a hydrocarbon chain and that comprises from 1 to 24 carbons; an aromatic substituent, with none, one or more substituents in the ring, and that comprises a hydrocarbon chain of between 6 and 18 carbons. R₄ is an oxygenated substituent of alcohol type (H₂C-OH) or alkoxide (H₂C-OR, where R is an alkyl or aryl group), and R₂ and R₃ are substituents identical or different from each other, and are indistinctly selected from hydrogen, oxygenated substituent of alcohol type (H₂C-OH), alkoxide (H₂C-oR, R being alkyl or aryl group), carboxyl (-COOH), carboxylate (COOR, where R is an alkyl or aryl group), an aliphatic or aromatic oxygenated heterocycle with 4 to 12 C atoms, substituted or unsubstituted, linear or branched, substituted or unsubstituted alkyl having 1 to 24 C atoms; cyclic alkyl with 4 to 24 C atoms, substituted or unsubstituted; or substituted or unsubstituted aryl with 6 to 18 C atoms.

According to the process of the present invention the tetrahydro-furfuryl ethers compounds synthesized are compounds having one or more tetrahydro-furan rings in its structure, being in our case preferably a mono-tetrahydro-furan compound. Said mono-tetrahydro-furan compound is preferably selected from 2-iso-propoxymethyl-tetrahydro-furfuryl ether, 2-sec-butoxymethyl-tetrahydro-furfuryl ether, 2-(2-hexoxy)-methyl-tetrahydro-furfuryl ether, 2-(2-octoxy)-methyl-tetrahydro-furfuryl ether, 2-iso-propoxymethyl-5-hydroxymethyl-tetrahydro-furfuryl ether, 2-sec-butoxymethyl -5-hydroxymethyl-tetrahydro-furfuryl ether, 2-(2-hexoxy) methyl-5-hydroxymethyl-tetrahydro-furfuryl ether, 2-(2-octoxy)-methyl-5-hydroxymethyl-tetrahydro-furfuryl ether, 2-iso-propoxymethyl-5-methoxymethyl-tetrahydro-furfuryl ether, 2-sec-butoxymethyl-5-methoxymethyl-tetrahydro-furfuryl ether, 2-(2-hexoxy) methyl-5-methoxymethyl-tetrahydro-furfuryl ether, 2-(2-octoxy) methyl-5-methoxymethyl tetrahydro-furfuryl ether, 2-iso-propoxymethyl-5-ethoxymethyl-tetrahydro-furfuryl ether, 2-sec-butoxymethyl-5-ethoxymethyl-tetrahydro-furfuryl ether, 2-(2-hexoxy) methyl-5-etoximetil-tetrahydro-furfuryl ether, 2-(2-octoxy)-methyl-5-ethoxymethyl-tetrahydro-furfuryl ether, and combinations thereof.

According to one embodiment of the present invention, the compounds that contain furan rings are preferably mono-furan derivatives, containing at least one or more oxygenated substituents of formyl type (-HC=O), and they may also contain one or more oxygenated substituents of alcohol type (H₂C-OH), alkoxide (H₂C-OR, where R is an alkyl or aryl group), carboxyl (-COOH), carboxylate (COOR, where R is an alkyl or aryl group); one or more substituents of aliphatic or aromatic oxygenated heterocycle type, substituted or unsubstituted, and that comprise a hydrocarbon chain of between 4 and 12 carbon atoms, one or more linear or cyclic aliphatic hydrocarbon substituents, with none, one or more chain branches, and that comprise a hydrocarbon chain of between 1 and 24 carbon atoms; one or more aromatic substituents with none, one or more substituents in the ring, and that comprise a hydrocarbon chain of between 6 and 18 carbons. Examples of these furan compounds are for example furfural, 5-hydroxymethyl furfural, 5-methoxymethyl furfural, 5-ethoxymethyl furfural, furan-2,5-di-carbaldehyde, furan-2,5-dicarboxylic acid, dimethyl furan-2,5-dicarboxylate, diethyl furan-2,5-di-carboxylate among others, without being limited to the examples.

According to one embodiment of the invention compounds that contain furan rings may have the general formula: 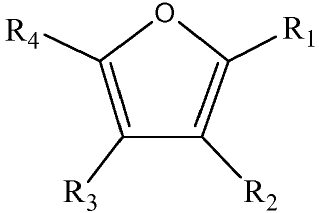 where R₁ is a formyl group (-HC= O) and R₂, R₃ and R₄ are identical or different substituents, and are indistinctly selected from: hydrogen, oxygenated substituent of alcohol type (H₂C-OH), alkoxide (H₂C-OR, where R is an alkyl or aryl group), carboxyl (-COOH), carboxylate (COOR, where R is an alkyl or aryl group), aliphatic or aromatic oxygenated heterocycle with 4 to 12 carbon atoms, substituted or unsubstituted; alkyl having 1 to 24 C atoms, linear or branched, substituted or unsubstituted; cyclic alkyl having 4 to 24 C atoms, substituted or unsubstituted; or aryl having 6 to 18 C atoms, substituted or unsubstituted.

According to one embodiment of the present invention, the compounds containing furan rings can have the general formula: 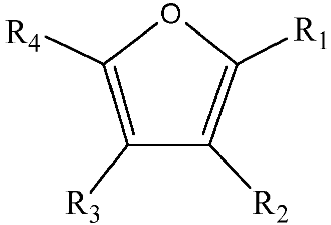 wherein R₁ is a formyl group (-HC=O), R₄ is an oxygenated substituent of alcohol type (H₂C-OH) or ether (H₂C -OR, where R is an alkyl or aryl group), and R₂ and R₃ are substituents identical or different from each other, and are indistinctly selected from: hydrogen, oxygenated substituent of alcohol type (H₂C-OH), alkoxy (H₂C-oR, where R is an alkyl or aryl group), carboxyl (-COOH), carboxylate (COOR, R being an alkyl or aryl group), oxygenated aliphatic or aromatic heterocycle with 4 to 12 C atoms, substituted or unsubstituted; alkyl having 1 to 24 C atoms, linear or branched, substituted or unsubstituted; cyclic alkyl with 4 to 24 C atoms, substituted or unsubstituted; or aryl with 6 to 18 C atoms, substituted or unsubstituted.

According to the process of the present invention the furan compounds selected are compounds having one or more furan rings in its structure, being in our case preferably a mono-furan compound. Said mono-furan compound is preferably selected from furfural, 5-hydroxymethyl furfural, 5-methoxymethyl furfural, 5-ethoxymethyl furfural, and combinations thereof.

According to one embodiment of the present invention, the alcohols used are preferably aliphatic, linear or cyclic primary or secondary alcohols, with none, one or more chain branches, and that comprise a hydrocarbon chain of between 1 and 24 carbon atoms; or aromatic primary or secondary alcohols, with none, one or more substituents on the ring and that comprise a hydrocarbon chain comprising between 6 and 18 carbon atoms.

Examples of these primary or secondary alcohols are for example methanol, ethanol, n-propanol, iso-propanol, n-butanol, 2-butanol, n-pentanol, 2-pentanol, n-hexanol, 2-hexanol, n-octanol, 2-octanol, n-decanol, 2-decanol, n-dodecanol, 2-dodecanol, cyclohexanol, benzyl alcohol, 2-phenylethanol, 1-phenyl-ethanol, 3-phenyl-1-propanol, 1-phenyl-1-propanol, 3-phenyl-2-propanol, without these being limiting examples.

According to one embodiment of the present invention, the alcohols used can have the general formula: 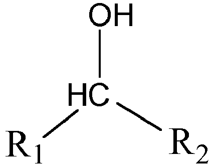 wherein R₁ and R₂ are identical or different substituents, and are indistinctly selected from: hydrogen, alkyl having 1 to 24 carbon atoms, linear or branched, substituted or unsubstituted; cyclic alkyl with 4 to 24 C atoms, substituted or unsubstituted; or aryl with 6 to 18 C atoms, substituted or unsubstituted.

According to the process of the present invention the selected alcohols are primary or secondary alcohols, linear or cyclic, aliphatic or aromatic alcohols, in our case being preferably an aliphatic primary or secondary alcohol. Said primary or secondary alcohol is preferably selected from ethanol, n-propanol, 2-propanol, n-butanol, 2-butanol, n-pentanol, 2-pentanol, n-hexanol, 2-hexanol, n-octanol, 2-octanol, n-decanol, 2-decanol, n-dodecanol, 2-dodecanol, and combinations thereof.

According to a preferred embodiment, the alcohol is selected from: an aliphatic primary alcohol having 2 to 12 carbon atoms, an aliphatic secondary alcohol having 2 to 12 carbon atoms, and combinations thereof.

According to another preferred embodiment of the present invention, the furan compound is furfural or 5-hydroxymethyl furfural, or combinations thereof, and the alcohol is 2-butanol.

According to another preferred embodiment, the furan compound is furfural or 5-hydroxymethyl furfural, or combinations thereof, and the alcohol is 2-octanol.

As already mentioned above, the etherification reaction is preferably carried out in the absence of hydrogen and the reduction reaction is preferably carried out in the presence of hydrogen. According to the process of the present invention the hydrogen source may be selected from pure molecular hydrogen, nitrogen enriched with hydrogen, argon enriched with hydrogen or a gas mixture comprising hydrogen and nitrogen combinations thereof. Said gaseous mixture may comprise two or more gases. For example, hydrogen or N₂ enriched with hydrogen, or hydrogen-enriched Ar, or mixtures thereof can be used, without being limited to the examples. The amount of hydrogen and the source selected will depend on the type of reactor and the specific reaction conditions of the process. The amount of hydrogen present in the reactive medium will always be the initial quantity of reagents used, and will depend on the temperature and pressure in the reactor.

As catalyst for the process of consecutive etherification / reduction described herein, at least one catalyst can be used, selected from:
a) a metallic catalyst "CAT A" comprising one or more noble metals, or one or more transition metals, or one or more of its salts or complexes, and combinations thereof, with the aforementioned "CAT A" being supported, or included in a carbonaceous type solid or in the structure of an inorganic matrix;
b) a metallic catalyst "CAT B" comprising one or more transition metals, their salts or complexes, included within or supported on an inorganic matrix structure;
c) a metallic catalyst "CAT C" comprising at least one noble metal and one or more transition metals, or one or more of its salts or complexes, and combinations thereof, said "CAT C" supported, or included in the structure of an inorganic matrix;
d) combinations thereof.

According to a particular embodiment, as a catalyst for the process of consecutive etherification / reduction described herein, a metallic catalyst "CAT A" containing at least one noble metal or a salt thereof can be used, such as Au, Pd, Ag, Pt, Ru, Re, Rh, and combinations thereof, supported on, or included within, the structure of a carbonaceous type solid or inorganic solid matrix, such as amorphous solids of carbon type, active carbons, graphenes, carbon nitrides, metal oxides, mixed metal oxides, or of the microporous molecular sieves types, mesoporous molecular sieves and combinations thereof. Preferably, the metallic catalyst "CAT A" contains at least one metal from among Ru, Pd, Pt, Rh, and combinations thereof, in which one of them is preferably Ru.

According to a particular embodiment, the transition metal in the "CAT A" catalyst is selected from Ti, Zr, Zn, Cu, Co, Mn, Mo, V, Ni, Fe, Al, and combinations thereof.

In another particular case of the process of consecutive etherification / reduction described herein, the metallic catalyst "CAT A" may consists of a compound selected from a salt and a transition metal complex, said salt or complex being supported on, or included in, the structure of a solid or inorganic matrix, such as amorphous solids, or of microporous molecular sieve types, mesoporous molecular sieves and combinations thereof. In the supported catalyst, said transition metal may be a metal of groups Ib, IIb, IVb, Vb, VIb, VIIb and VIII of the periodic table, such as Cu, Co, Mn, Ni, Fe, Ce and combinations of the same.

Non-limiting examples of amorphous carbonaceous solids used include: carbons, active carbons, graphene, carbon nitrides, among others

Non-limiting examples of inorganic amorphous solid matrices used one could mention: silica, alumina, titania, ceria, yttria, Fe oxides, silica-alumina, silica-ceria, and in general mixed oxides of metals and/or transition metals such as Cu, Zn, Ti, Ce, Mn, V, Ni, Fe, Sn, Mo, among others.
According to a particular embodiment of the present invention, the inorganic matrix may be selected from: silica, alumina, ceria, yttria, titania, Fe₂O₃, silica-alumina, silica-ceria, one or more mixed oxides of alkaline earth metals, one or more transition metal oxides.

According to another particular embodiment, said inorganic matrix is an amorphous siliceous material comprising Si and an element selected from Sn, Zr, Ti, Ga, Ta, Al, or combinations thereof.

Non-limiting examples of inorganic amorphous solid matrices used could include solids made up of oxides of alkaline earth metals (MgO, CaO, BaO), preferably MgO, together with oxides of other metals, and in general mixed oxides derived from anionic clays such as layered double hydroxides of the hydrotalcite type (Mg/Al).

Non-limiting examples of microporous solid matrices used could include: microporous silicates, including pure silica zeolites, microporous aluminosilicate including Al-zeolites, microporous metal-silicates including Me-zeolites, microporous alumino-phosphate (ALPOs, APO's, etc.), microporous alumino-phosphates containing metals (Me-APO's), microporous silico-aluminophosphates (SAPO's, TAPSO's, etc.). Layered materials such as clays and pillared clays, of the bentonite, montmorillonite type, etc, can also be used as microporous inorganic matrices.

Non-limiting examples of mesoporous solid matrices employed could include: silicates, alumino-silicates, and generally mesoporous metallo-silicate of hexagonal or cubic structure, such as MCM-41, MCM-48, SBA-15, HMS, MSA, among others. Mesoporous materials obtained by delamination of laminar zeolitic precursors, such as ITQ-2 [A. Corma et al., Nature, 396, 353, 1998], ITQ-6 [A. Corma et al., Angew. Chem. Int. Ed., 39(8), 1499, 2000], among others, can also be used as mesoporous solid matrices.

According to a particular embodiment of the present invention, the carbonaceous solid CAT A has a surface area between 50 and 1200 m²/g and is a material selected from: carbons, active carbons, carbon nanotubes, graphense, carbon nitrides, and combinations thereof.

According to another particular embodiment, the transition metal in the "CAT B" catalyst and "CAT C" catalyst is selected from Si and Sn, Zr, Ti, Ga, Ta, Al, or combinations thereof.

In another particular case of the process of consecutive etherification / reduction described in the present invention, a metallic catalyst "CAT B" can be used, which may consist of a microporous molecular sieve, a mesoporous molecular sieve, or even amorphous siliceous materials containing Si and an element selected from Sn, Zr, Ti, Ta, Ga, Al, or combinations thereof.

In a particular case of the present invention, the microporous molecular sieve employed as catalyst is a zeolite Beta type in which a portion of the silicon atoms are replaced by an element selected from tin, zirconium, titanium, aluminum, gallium, tantalum and combinations thereof. A microporous molecular sieve of Beta zeolite type can have the following empirical formula in its calcined and anhydrous state:

y(A_{1/n} ⁿ⁺ XO₂) : t TO₂ : SiO₂: x SnO₂

in which,
- X represents at least one trivalent element, preferably selected from Al, or Ga, Ta or, or combinations thereof,
- "y" is a number comprised between 0 and 0.2,
- A represents a mono-, di-, or trivalent cation, or combinations thereof,
- n = 1, 2 or 3,
- T represents at least one tetravalent element other than Si and Sn, preferably selected from Ti or Zr, or combinations thereof,
- "t" is a number comprised between 0 and 0.2,
- "x" is a number comprised between 0 and 0.2, and preferably between 0.001 and 0.1.

The microporous materials of Beta zeolite type are prepared by a process of hydrothermal crystallization in a reaction medium comprising a silicon source, a source of tin and/or zirconium, optionally another metal (M), one structure directing agent, a mobilizing agent as OH⁻ or F⁻, optionally hydrogen peroxide and water.

Numerous sources of silicon with said element in oxidation state +4 can be used. Non-limiting examples may be mentioned: silica under the form of hydrogels, aerogels, xerogels, in colloidal suspensions, silicas obtained by precipitation from solutions of soluble silicates or from the hydrolysis of siliceous esters such as Si(OCH₃)₄ and Si(OC₂H₅)₄. Hydrolysable tetravalent silicon compounds can also be used such as silicon halides, or analogs. The source of silicon preferably selected are alkyl silicates, and more preferably tetra-ethyl silicate. Non-limiting examples of sources of tin are: the tin halides, and preferably SnCl₄, tin alkoxides, metallic tin, alkaline stannates or alkaline earth metal stannates, and compounds of alkyl-tin type.

Non-limiting examples of zirconium sources are: the oxides and hydroxides of zirconium, crystalline or amorphous, hydrolysable zirconium compounds such as zirconium halides, derivatives of alkyl zirconate type, soluble zirconium salts, among others.

Non-limiting examples of structure directing agents that may be mentioned are: those of ionic type, such as tetraethylammonium ions, dialkyldibenzylammonium, bis-piperidinium such as 4,4'-trimethylen-bis-(N-benzyl-N-methylpiperidinium). These ions can be in the form of hydroxide or halide compounds type, preferably chlorides or bromides. Also as examples of structure directing agents compounds of aza-polycyclic type, such as 1,4-diazabicyclo-2,2,2-octane, can also be cited.
According to the process of the present invention, these materials of microporous molecular sieve type may contain Si, and at least one of the following elements: Sn, Zr, Ti, Ta, Ga, Al. Si-C bonds can be introduced in the material, for example, by silylation process making up an organic-inorganic composite. Said organic-inorganic composite further comprises Si, at least one element selected from Sn, Zr, Ti, Ta, Ga, Al, and may also contain some silicon atoms linked to carbon, produced, for example, by a method comprising a silylation step during synthesis, or by a process comprising a post-synthesis silylation stage.

Such organic-inorganic composites can be a microporous molecular sieve that comprises Si and further comprises at least one element selected from Sn, Zr, Ti, Ta, Ga, Al, and silicon bonded to carbon, or they can consist of amorphous inorganic siliceous solids chemically combined with one element selected from Sn, Zr, Ti, Ta, Ga, Al, or combinations thereof, in proportions of between 0.2 and 8% by weight of an element selected from Sn, Zr, Ti, Ta, Ga, Al or combinations thereof, as an oxide form on the total catalyst, and which contain silicon bonded to carbon.

Among these microporous materials one can mention molecular sieves which have a structure corresponding to a zeolite selected from Beta zeolite, Mordenite and ITQ-16 [structure Corma et al, WO 2002030821 Al.; WO 2002064503 Al; and Chem. Commun., 18, 1720, 2001], without these being limiting examples. In the particular case that the microporous material possesses a crystal structure of Beta type, it can be selected from among crystal structures of a Beta zeolite, a polymorph of Beta zeolite, and combinations thereof.

According to a particular embodiment, in said Beta zeolite, the Si atoms are partially replaced by Sn, or Zr, Ti, Ga, or Ta, or Al, or combinations thereof.

In a particular case of the present invention, the mesoporous molecular sieve employed as catalyst is a material of the MCM-41 type in which a portion of the silicon atoms are replaced by an element selected from tin, zirconium, titanium, aluminum, gallium, tantalum and combinations thereof. The precursor of the mesoporous molecular sieve of the MCM-41 type used as catalyst can have the chemical formula:

y(Aⁿ⁺_{1/n} XO₂ ) : t TO₂ : (1-m)S,O₂ : x SnO₂ : m R₍₄₋ₚ₎ SiO_{P/2} : s S

wherein:
- A represents one or more mono-, di- or trivalent compensating cations, or combinations thereof,
- X represents at least one trivalent element, preferably selected from Al, Ga, or Ta, or combinations thereof
- "y" is a number comprised between 0 and 0.2,
- n = 1, 2 or 3,
- T represents at least one tetravalent element other than Si and Sn, preferably selected from Ti or Zr, or combinations thereof,
- "t" is a number comprised between 0 and 1, and preferably between 0 and 0.2,
- "x" is comprised between 0 and 0.2,
- S represents an organic compound,
- "s" is a number that can vary between 0 and 0.5.
- "m" is a number comprised between 10-6 and 0.66,
- "p" is a number comprised between 3 and 1,
- and where R is an alkyl group, aromatic or a combination thereof derived from the silylation agent that contains the SiC bonds.

The organic compound corresponding to the S group is extracted by chemical means and the mesoporous molecular sieve undergoes a post-synthesis treatment with a silylation agent giving rise to the formation of new Si-C bonds.

According to the process of the present invention, these materials of the mesoporous molecular sieve type may contain Si and an element selected from Sn, Zr, Ti, Ta, Ga, Al, or combinations thereof, and Si-C bonds, forming a organic-inorganic composite. Said organic-inorganic composite comprising at least Si and one element selected from Sn, Zr, Ti, Ta, Ga, Al, or combinations thereof, and silicon bonded to carbon is obtained by a process which comprises a silylation stage during synthesis or by a process comprising a post-synthesis silylation stage.

Such organic-inorganic composites can be a mesoporous molecular sieve comprising Si, and further comprising at least one element selected from Sn, Zr, Ti, Ta, Ga, Al, and silicon bonded to carbon, or can consist of amorphous inorganic siliceous solids chemically combined with one element selected from among Sn, Zr, Ti, Ta, Ga, Al, or combinations thereof, in proportions of between 0.2 and 8% by weight of an element selected from among Sn, Zr, Ti, Ta, Ga, Al or combinations thereof, as an oxide over the total catalyst, and which contain silicon bonded to carbon.

According to a particular embodiment, in the mesoporous molecular sieve the Si atoms are partially replaced by Sn, or Zr, Ti, Ga, or Ta, or Al, or combinations thereof.

Among these mesoporous solid materials, ordered mesoporous materials may be mentioned such as MCM-41, MCM-48, SBA-15, HMS, and other amorphous ones, such as amorphous silica. Tin, zirconium, titanium, tantalum, gallium, or combinations thereof, are introduced in the synthesis stage, or in a treatment after the synthesis. Furthermore, said materials may have organic groups anchored on their surface.

In another particular case of the process of consecutive etherification/reduction described in the present invention, a metallic catalyst "CAT C", which may consist of a noble metal or a salt thereof, such as Au, Pd, Ag, Pt, Ru, Re, Rh, or combinations thereof, can be used, supported on, or included within, the structure of an inorganic solid of microporous molecular sieve type or mesoporous molecular sieve, or even amorphous siliceous materials, containing Si, and at least one transition metal selected among Sn, Zr, Ti, Ta, Ga, Al. Preferably, the metallic catalyst "CAT C" contains Pd, Pt, Ru, Rh, or combinations thereof, and more preferably Pt or Pt in combination with a second metal. Preferably, to support the noble metal or metals on the catalyst "CAT C", microporous molecular sieves are used containing Si, and further containing at least one transition metal selected from Sn, Zr, Ti, Ta, Ga, Al, and more preferably microporous structures of Beta zeolite type.

A microporous molecular sieve of Beta zeolite type used as inorganic matrix of metallic catalyst "CAT C" can have the following empirical formula in its calcined and anhydrous state:

y(A_{1/n}ⁿ⁺ XO₂) : t TO₂ : SiO₂: x SnO₂

wherein,
- X represents at least a trivalent element, preferably selected among Al, or Ga, or Ta, or combinations thereof,
- "y" is a number comprised between 0 and 0.2,
- A represents a mono-, di-, or trivalent cation, or combinations thereof,
- n = 1, 2 or 3,
- T represents at least one tetravalent element other than Si and Sn, preferably selected from Ti or Zr, or combinations thereof,
- "t" is a number comprised between 0 and 0.2,
- "x" is a number comprised between 0 and 0.2, and preferably between 0,001 and 0.1.

The microporous materials of Beta zeolite type are prepared by a process of hydrothermal crystallization in a reaction medium comprising a silicon source, a source of tin and/or zirconium, optionally another metal (M), one structure directing agent, a mobilizing agent as OH- or F-, optionally hydrogen peroxide and water.

Numerous sources of silicon with said element in +4 oxidation state can be used. Non-limiting examples may be mentioned: silica under the form of hydrogels, aerogels, xerogels, in colloidal suspensions, silicas obtained by precipitation from solutions of soluble silicates or hydrolysis of siliceous esters such as Si(OCH₃)₄ and Si (OC₂H₅)₄. Hydrolysable tetravalent silicon compounds can also be used such as silicon halides, or analogs. The silicon sources preferably selected are alkyl silicates, and more preferably tetra-ethyl silicate.

Non-limiting examples of tin sources are: the tin halides, and among them, those preferably including SnCl₄, tin alkoxides, metallic tin, alkaline or alkaline earth metal stannates, and compounds of alkyl-tin type.

Non-limiting examples of zirconium sources are: zirconium oxides and hydroxides, crystalline or amorphous, hydrolysable zirconium compounds such as zirconium halides, derivatives of alkyl-zirconate type, soluble zirconium salts, among others.

Non-limiting examples of structure directing agents which may be mentioned: those of ionic type such as tetraethylammonium ions, dialkyldibenzylamonium, bis-piperidinium such as 4,4'-trimethylen-bis-(N-benzyl-N-methylpiperidinium). These ions can be in the form of hydroxide or halide compounds type, preferably chlorides or bromides. Compounds of aza-polycyclic type, such as 1,4-diazabicyclo-2,2,2-octane can also be cited as structure directing agents.

Non-limiting examples of solid microporous matrices used could include: microporous silicates including pure silica zeolites, microporous aluminosilicate including Al-zeolites, microporous metal-silicates including Me-zeolites, microporous alumino-phosphate (ALPOs, APOs, etc.), microporous alumino-phosphates containing metals (Me-APOs), micro-porous silico-aluminophosphates (SAPO's, TAPSO's, etc.).

According to a particular embodiment, the inorganic matrix described herein is an amorphous material selected from one or more metal oxides, one or more mixed metal oxides, and combinations thereof.

Non-limiting examples of meso-porous solid matrices employed could include: silicates, alumino-silicates, and in general mesoporous metal-silicate with hexagonal or cubic structure, such as MCM-41, MCM-48, SBA-15, HMS, MSA, among others. Mesoporous materials obtained by delamination of laminar zeolitic precursors, such as ITQ-2, ITQ-6, among others, can also be used as mesoporous solid matrices.

In the particular case of metallic catalyst "CAT C" described herein, the integration, or supported, or inclusion of a noble metal or a salt thereof, such as Au, Pd, Ag, Pt, Ru, Re, Rh, or combinations thereof, in the structure of an inorganic solid matrix can be carried out during the synthesis stage of said inorganic matrix or through post-synthesis stages. These post-synthesis stages may be selected from: wet impregnation, incipient wetness impregnation (or pore volume), precipitation, deposition, precipitation-deposition, and combinations thereof. To carry out this stage of incorporation, support, or post-synthesis inclusion, the corresponding sources of metals or metal salts to be incorporated are previously, and in adequate quantity, dissolved in a solvent. Non-limiting examples of suitable solvents for this post-synthesis metal incorporation may include: water, methanol, ethanol, iso-propanol, 1-butanol, 2-butanol, and mixtures thereof. For this step of post-synthesis incorporation solvents as ethyl ether, tert-butyl methyl ether, acetone, 2-butanone, methyl iso-butyl ketone, ethyl acetate, acetonitrile, methylene chloride, chloroform, can also be used without being limited to these examples.

The process for the consecutive etherification/reduction of compounds of mono-furfuryl derivative type in the presence of alcohols is characterized in that the etherification/reduction reactions can be carried out consecutively in cascade and in the same reactor, or in two steps in independent reactors.

In case that the etherification/reduction reactions are carried out in consecutive manner in cascade, and in a single reactor ("one pot"), the reactor used may be a batch reactor, a continuous stirred tank reactor (CSTR), in a continuous fixed bed reactor, in a fluidized bed reactor, or in an boiling bed reactor.

In the case that the etherification/reduction reactions are carried out independently, the reactor used for the etherification reaction may be a batch reactor, a continuous stirred tank reactor (CSTR), a continuous fixed bed reactor, in a fluidized bed reactor, or a boiling bed reactor.

In the case that the etherification/reduction reactions are carried out independently, the reactor used for the reduction reaction may be a batch reactor, a continuous stirred tank reactor (CSTR), a semi-continuous reactor, or a continuous fixed bed reactor.

According to a particular embodiment of the present invention, the consecutive etherification/reduction reactions of furanic compounds can be preferably carried out with a weight ratio of alcohol to furan compound from 2 to 200, a preferred temperature between 20 and 250 °C in a reaction time preferably comprised between 2 minutes and 200 hours and a total pressure in the system preferably comprised between atmospheric pressure and 50 bar.

In one embodiment of the present invention the process of consecutive etherification/reduction of compounds of mono-furfuryl derivative type in the presence of alcohols is performed by a cascade reaction by contacting a reaction mixture containing one or more mono-furfuryl derivatives, a hydrogen source (preferably H₂ or N₂ enriched with H₂), one or more alcohols with one or more metal catalysts selected among "CAT A", "CAT B", "CAT C", or a mixture of them, in a pressure range that can vary from atmospheric pressure up to 50 bar, at a temperature comprised between 20 and 250 °C, for reaction times which may vary between 2 minutes and 200 hours depending on the catalyst and reaction conditions employed.

In the process of the present invention carried out in a cascade reaction, the weight ratio of the mono-furfuryl derivative to the catalyst is comprised between 1 and 200, and more preferably between 2 and 100. The weight ratio between alcohol and mono-furfuryl derivative may be comprised between 2 and 200.

In case that the etherification/reduction is performed consecutively in cascade in a batch reactor, the weight ratio of the mono-furfuryl derivative to the catalyst is preferably comprised between 2 and 200, more preferably between 2 and 100. The weight ratio of the mono-alcohol and furfuryl derivative may be preferably comprised between 2 and 200, more preferably between 2 and 100. The process temperature in a batch reactor is preferably comprised between 20 and 250 °C, more preferably between 40 and 200 °C. The reaction time in a discontinuous reactor preferably ranges between 2 minutes and 36 hours. The etherification/reduction reaction, when carried out in a batch reactor, is performed at a total pressure in the system preferably between atmospheric pressure and 50 bar.

In case the etherification / reduction is performed consecutively in cascade in a continuous reactor, the weight ratio of the mono-furfuryl derivative to the catalyst is preferably comprised between 1 and 200. The weight ratio between alcohol and mono-furfuryl derivative may be preferably comprised between 2 and 200, more preferably between 2 and 100. The process temperature in a continuous reactor is preferably comprised between 20 and 250°C, more preferably between 20 and 200°C. The reaction time in a discontinuous reactor preferably ranges between 2 minutes and 200 hours. The etherification/reduction reaction, when carried out in a continuous reactor, is performed at a total pressure in the system preferably comprised between atmospheric pressure and 50 bar.

In one embodiment of the present invention, the process of etherification/reduction of compounds of mono-furfuryl derivative type in the presence of alcohols is accomplished by independent reactions by contacting a reaction mixture containing one or more mono-furfuryl derivatives, one or more alcohols, with a metallic catalyst "CAT A", or a metallic catalyst "CAT B", or a metallic catalyst "CAT C", or a mixture thereof, in presence or absence of a hydrogen source (preferably H₂ or N₂ enriched with H₂, or only N₂) in a range of pressures that can vary from atmospheric pressure up to 50 bar, at a temperature between 10 and 250°C, for reaction times which may vary between 2 minutes and 1000 hours depending on the catalyst and reaction conditions employed.

In the process of the present invention carried out in two separate reactions, the weight ratio of the mono-furfuryl derivative to the catalyst is comprised between 1 and 200, preferably between 2 and 100. The weight ratio between alcohol and mono-furfuril derivative may preferably be comprised between 2 and 200.

In case the etherification/reduction process is performed in two independent reactions, and the etherification reaction is carried out in a batch reactor and in the absence of hydrogen, the weight ratio of the mono-furfuryl derivative to catalyst is preferably comprised beteen 2 and 200, more preferably from 2 to 100. The weight ratio between the alcohol and mono-furfuryl derivative may be preferably comprised between 2 and 200, more preferably between 2 and 100. The process temperature in the batch reactor is preferably between 20 and 250 °C, more preferably between 40 and 200 °C. The reaction time in a discontinuous reactor preferably ranges between 2 minutes and 36 hours. When the etherification reaction is carried out in a batch reactor, it is performed at a total pressure in the system preferably comprised between atmospheric pressure and 50 bar, and more preferably between atmospheric pressure and 15 bar.

In case the etherification/reduction process is performed in two independent reactions, and the etherification reaction is performed in a continuous reactor in the absence of hydrogen, the weight ratio of the mono-furfuryl derivative to catalyst is preferably comprised between 1 and 200, more preferably from 2 to 100. The weight ratio between the alcohol and mono-furfuryl derivative may be preferably comprised between 2 and 200, more preferably between 2 and 100. The process temperature in the continuous reactor is preferably comprised between 20 and 250 °C, more preferably between 20 and 200 °C. The reaction time in a discontinuous reactor preferably ranges between 2 minutes and 200 hours. When the etherification reaction is carried out in a continuous reactor, it is performed at a total pressure in the system preferably comprised between atmospheric pressure and 50 bar, and more preferably between atmospheric pressure and 15 bar.

In case the etherification/reduction process is performed in two independent reactions, and the reduction reaction is performed in a batch reactor in the presence of hydrogen, the weight ratio of the mono-furfuryl derivative to the catalyst is preferably comprised between 2 and 200, more preferably from 2 to 100. The weight ratio between the alcohol and mono-furfuryl derivative may be preferably comprised between 2 and 200, more preferably between 2 and 100. The process temperature in a reactor batch is preferably between 20 and 250 °C, more preferably between 40 and 200 °C. The reaction time in a discontinuous reactor preferably ranges between 2 minutes and 36 hours. When the etherification reaction is carried out in a batch reactor is performed at a total pressure in the system preferably comprised between atmospheric pressure and 50 bar. In case the etherification/reduction process is performed in two independent reactions, and the etherification reaction is performed in a continuous reactor, the weight ratio of the mono-furfuryl derivative to the catalyst is preferably comprised between 1 and 500, more preferably between 2 and 200. The weight ratio of the alcohol and mono-furfuryl derivative may be preferably comprised between 2 and 200, more preferably between 2 and 100. The process temperature in a continuous reactor is preferably comprised between 20 and 250 °C, more preferably between 20 and 200 °C. The reaction time in a discontinuous reactor preferably ranges between 2 minutes and 200 hours. When the etherification reaction is carried out in a continuous reactor, it is performed at a total pressure in the system preferably comprised between atmospheric pressure and 50 bar.

The present invention describes a process, preferably in a cascade reaction type, for consecutive etherification/reduction of a compound containing one or more furan rings, preferably a mono-furfuryl derivative, and more preferably 5-hydroxymethyl furfural and furfural, in the presence of one or more alcohols and one or more catalysts, and optionally hydrogen. Through the process of the present invention the corresponding tetrahydro-furfuryl ethers can be obtained with excellent yields, and also mixtures of furfuryl ethers and tetrahydro-furfuryl ethers, which can be easily separated by fractioned distillation, or be used in mixtures of compositions suitable as additives for diesel.

The process described in the present invention has significant competitive advantages when compared to other processes already described. First, this process allows the efficient and highly selective preparation of the tetrahydro-furfuryl ethers derivative type, starting from furfuryl derivatives and alcohols, with yields close to 90%, and overall yields to the corresponding final product close to 80%. Second, the process can be carried out by a cascade reaction ("one-pot") without a solvent and at low H₂ pressures (<10bar) and temperatures (≤130 ° C). Furthermore, the process can be carried out with a combination of heterogeneous solid catalysts of easy production and application in the reagent system, eliminating the risks and dangers of handling some hydrogenation catalysts, such as Ni/SiO₂.

Throughout the description and claims the word "comprise" and its variations are not intended to exclude other technical features, additives, components or steps. For those skilled in the art, other objects, advantages and features of the invention will arise partly from the description and partly from practice of the invention. The following examples illustrate the preparation of metal catalysts and the application thereof in the process of consecutive etherification/reduction of mono-furfuryl derivative in the presence of alcohols to obtain tetrahydro-furfuryl ethers, and are provided by way of illustration, and it is not intended to be limiting of the present invention.

### Examples

### Example 1: This example illustrates the synthesis of a Sn-Beta zeolite (CAT B).

30.00 g of tetraethylorthosilicate (TEOS) and 32,99 g of a tetraethylammonium hydroxide solution (TEAOH, 35 wt%) are mixed in a container. After 90 minutes, a solution of 0.43 g of SnCl₄·5H₂O (98%) in 2.75 g of water is added, and the mixture is stirred until the ethanol formed by the hydrolysis of the TEOS evaporates. To this clear solution 3.20 g of HF (48 wt%) are added, yielding a thick paste. Finally, a suspension of 0.36 g of Beta zeolite seeds is added (prepared as described in Spanish patent P9501552) in 1.75 g of water. The final gel composition obtained detailed in the following formula:

SiO₂: 0,54 TEAOH: 0,008 SnO₂: 0,54 HF: 7,5 H₂O

The gel is placed in a stainless steel autoclave with Teflon coated, inside, heated to 140 °C and reacted for 11 days with stirring. After 11 days, the product is recovered by filtration, revealing through an analysis by X-ray diffraction the structure of Beta zeolite with a crystallinity of about 95%. Subsequent chemical analysis show that the product contains 1.62% by weight of tin. The product was calcined at 580 °C for 3 hours and maintained its crystallinity.

### Example 2: This example illustrates the synthesis of a Zr-Beta (CAT B).

In a container 37.50 g of tetraethylorthosilicate (TEOS) are mixed with 41.23 g of tetraethylammonium hydroxide (TEAOH, 35 wt%). After 90 minutes, a solution of 0.49 g of ZrOCl₂·8H₂O (98%) is added in 3.50 g of water, and the mixture is stirred until the ethanol formed by the hydrolysis of the TEOS evaporates. To this clear solution 4.08 g of HF (48 wt%), is added, yielding a thick paste. Finally, a suspension of 0.45 g of Beta zeolite seeds is added (prepared as described in Spanish Patent P9501552) in 2.50 g of water. The final gel composition obtained is detailed in the following formula:

SiO₂: 0,54 TEAOH: 0.008 ZrO₂: 0,54 HF: 7,5 H₂O

The gel is placed in a stainless steel autoclave with Teflon coated inside, heated to 140 ° C and reacted for 14 days with stirring. After 14 days, the product is recovered by filtration, revealing through an analysis by X-ray diffraction that it has the structure of Beta zeolite with a crystallinity of about 95%. Subsequent chemical analysis show that the product contains 1.15% by weight of zirconium. The product was calcined at 580 °C for 3 hours and maintained its crystallinity.

### Example 3: This example illustrates the synthesis of a Al-Beta zeolite (CAT B) with a molar ratio Si/Al = 50.

In a container, 20.00 g of tetraethylorthosilicate (TEOS), 21.80 g of a tetraethylammonium hydroxide solution (TEAOH, 35% in water), 0.39 g of Al iso-propoxide and 5.00 g of water are mixed. The resulting mixture is stirred until complete evaporation of the ethanol formed by the hydrolysis of TEOS. To this solution, 2.16 g of HF (48 wt%) is added, yielding a thick paste. Finally, a suspension of 0.25 g of Beta zeolite seeds is added (prepared as described in Spanish Patent P9501552) in 2.00 g of water. The final gel composition obtained is detailed in the following formula:

SiO₂: 0,54 TEAOH: 0,01 Al₂O₃: 0,54 HF: 7,5 H₂O.

The gel is placed in a stainless steel autoclave with Teflon coated inside, heated to 140 °C and reacted for 2 days with stirring. After 2 days, the product is recovered by filtration, revealing through an analysis by X-ray diffraction that it has the structure of Beta zeolite with a crystallinity near 100%. Subsequent chemical analysis show that the product has a molar ratio Si/Al = 50. The product was calcined at 580 °C for 3 hours and its crystallinity is >93% (crystal size ≈0,3 µm, micropore vol.= 0.19 cm³/g).

### Example 4: This example illustrates the synthesis of a Ga-Beta (CAT B) zeolite.

In a container, 20.00 g of tetraethylorthosilicate (TEOS) and 22.03 g of tetraethylammonium hydroxide (TEAOH, 35 wt%) are mixed. After 90 minutes, a solution of 0.42 g of Ga(NO₃)₃·10H₂O in 3.00 g of water is added, and the mixture stirred until the ethanol formed by the hydrolysis of the TEOS evaporates. To this clear solution 2.16 g of HF (48 wt%) are added, yielding a thick paste. Finally, a suspension of 0.25 g of Beta zeolite seeds is added (prepared as described in Spanish Patent P9501552) in 2.00 g of water. The final gel composition obtained is detailed in the following formula:

SiO₂: 0, 54 TEAOH: 0, 005 Ga₂O₃: 0, 54 HF: 7,5 H₂O

The gel is placed in a stainless steel autoclave with Teflon coated inside, heated to 140 °C and reacted for 7 days with stirring. After 7 days, the product is recovered by filtration, revealing through an analysis by X-ray diffraction that it has the structure of Beta zeolite with a crystallinity of about 100%. Subsequent chemical analysis show that the product contains 1.21% by weight of gallium. The product was calcined at 580 °C for 3 hours and maintained its crystallinity.

### Example 5: This example illustrates the synthesis of a Ta-Beta zeolite (CAT B).

In a container, 20.00 g of tetraethylorthosilicate (TEOS) and 0.39 g ethoxide Ta (V) are mixed. Then, 22.03 g of tetraethylammonium hydroxide (TEAOH, 35 wt%) and 2.00 g of water are added and the mixture is stirred until the ethanol formed by the hydrolysis of the TEOS evaporates. To this clear solution 2.16 g of HF (48 wt%) are added, yielding a thick paste. Finally, a suspension of 0.25 g of Beta zeolite seeds is added (prepared as described in Spanish Patent P9501552) in 2.00 g of water. The final gel composition obtained is detailed in the following formula:

SiO₂: 0,54 TEAOH: 0,005 Ta₂O₅: 0,54 HF: 4,0 H₂O

The gel is placed in a stainless steel autoclave with Teflon coated inside, heated to 140 °C and reacted for 14 days with stirring. After 14 days, the product is recovered by filtration, revealing through an analysis by X-ray diffraction that it has the structure of Beta zeolite with a crystallinity of about 85%. Subsequent chemical analysis show that the product contains 2.74% by weight of tantalum. The product was calcined at 580 °C for 3 hours and maintained its crystallinity.

### Example 6: This example illustrates the synthesis of a Beta Sn-Zr-zeolite (CAT B) with a molar ratio Si/Sn = 96 and a molar ratio Si/Zr = 266.

In a container 20.00 g of tetraethylorthosilicate (TEOS) and 22.08 g of a tetraethylammonium hydroxide solution (TEAOH, 35 wt%) are mixed. After 90 minutes, a solution of 0.28 g of SnCl₄·5H₂O (98%) and 0.13 g of ZrOCl 2-8h 2 0 (98%) in 7.00 g of water is added, and the mixture is stirred until the ethanol formed by the hydrolysis of the TEOS evaporates. To this solution, 2.16 g of HF (48 wt%) is added, yielding a thick paste. Finally, a suspension of 0.25 g of Beta zeolite seeds is added (prepared as described in Spanish Patent P9501552) in 2.00 g of water. The final gel composition obtained is detailed in the following formula:

SiO₂: 0,54 TEAOH: 0,008 SnO₂: 0,004 ZrO₂: 0,54 HF: 7,5 H₂O

The gel is placed in a stainless steel autoclave with Teflon coated inside, heated to 140 °C and reacted for 25 days with stirring. After 25 days, the product is recovered by filtration, revealing through an analysis by X-ray diffraction that it has the structure of Beta zeolite with a crystallinity of about 95%. Subsequent chemical analysis show that the product contains 1.97% by weight of tin and 0.55 wt% zirconium (molar ratio Sn/Zr = 2.77). The product was calcined at 580 °C for 3 hours and maintained its crystallinity.

### Example 7: This example illustrates the preparation of Pt/Sn-Beta (CAT C) material by incorporating 1.4% by weight of Pt in the Sn-Beta zeolite synthesized in Example 1.

A solution of 0.3033 g of H₂PtCl₆•6H₂O in 13.44 ml of water is prepared. Then, 0.70 ml of this solution were slowly added by incipient wetness impregnation method of 0.7034 g Sn-Beta, synthesized in Example 1 which are homogeneously dispersed into a flat-bottom vessel. The material obtained was dried in oven at 100 °C for 1 night and then calcined at 580 °C for 3 hours while keeping its crystallinity (> 90%). Subsequent chemical analysis show that the product contains 1.4% by weight of platinum. The solid material thus obtained is subjected to an activation process in a H₂ atmosphere at 350 °C for 3 hours for further use in catalytic experiments.

### Example 8: This example illustrates the use of the materials of Examples 1 to 5 as catalysts ("CAT B") in the direct etherification of furfuraldehyde with 2-butanol in a discontinuous or batch reactor.

In a two mouth glass reactor of 10 ml, one mouth connected to a condenser, and containing a magnetic bar, 100 mg of furfural, 1100 mg of 2-butanol and 50 mg of a catalyst are introduced as described in Examples 1 to 5 ("CAT B"). The second reactor mouth is closed by a septum system which allows taking samples at different time intervals. Next, the reaction temperature is raised to 100 °C, by immersing the reactor in a silicone bath with temperature control. The reaction mixture is stirred and samples are taken at various time intervals up to 7 hours of reaction. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product/moles of total products * 100) in each case. Thus the following results were obtained:

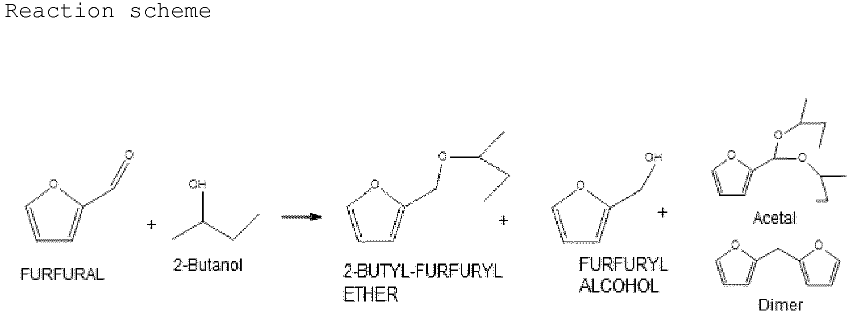

| **Catalyst** | | **Conversion (% Mol.)** | **Selectivity (% Mol.)** | | |
|---|---|---|---|---|---|
| **Example No** | **Type** | | **Furfuryl ether^{a}** | **Furfuryl Alcohol^{b}** | **Others^{c}** |
| 1 | Sn-Beta | 27.2 | 70.0 | 22.0 | 8.0 |
| 2 | Zr-Beta | 48.2 | 63.0 | 30.0 | 7.0 |
| 3 | Al-Beta | 1.1 | 0 | 0 | 100.0 |
| 4 | Ga-Beta | 2.1 | 0 | 0 | 100.0 |
| 5 | Ta-Beta | 25.0 | 17.0 | 83.0 | 0 |

| | | | | | |
|---|---|---|---|---|---|
| a-Furfuryl ether = 2-butyl-furfuryl ether.- b- Furfuryl Alcohol = 2-hydroxymethylfuran.- c- Others = Acetal + Dimer.- | | | | | |

### Example 9: This example illustrates the use of the materials of Examples 1 and 2 as catalysts ("CAT B") in the direct etherification of 5-hydroxymethylfurfural with 2-butanol in a discontinuous or batch reactor.

In a two mouth glass reactor of 10 ml, one mouth connected to a condenser, and containing a magnetic bar, 110 mg of 5-hydroxymethylfurfural, 3300 mg of 2-butanol and 50 mg of a catalyst as those described in Examples 1 and 2 ("CAT B"), are introduced. The second reactor mouth is closed by a septum system which allows taking samples at different time intervals. Next, the reaction temperature is raised to 100 °C, by immersing the reactor in a silicone bath with temperature control. The reaction mixture is stirred and samples are taken at various time intervals up to 7 hours of reaction. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:

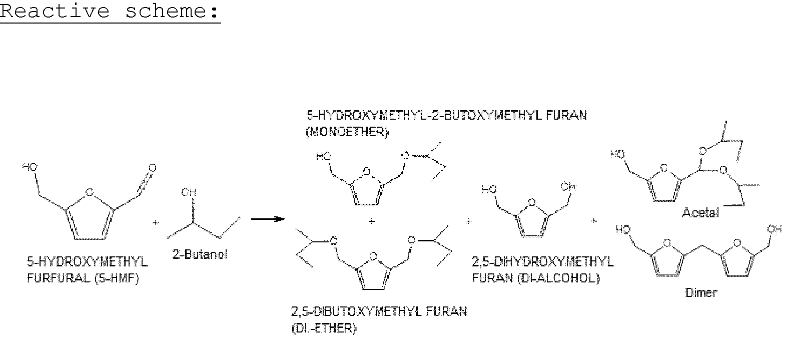

| **Catalyst** | | **Conversion (% Mol.)** | **Selectivity (% Mol.)** | | |
|---|---|---|---|---|---|
| **Example N°** | **Type** | | **Mono- y DiEther^{a}** | **Di-Alcohol^{b}** | **Others ^{c}** |
| 1 | Sn-Beta | 19.0 | 84.0 | 11.0 | 5.0 |
| 2 | Zr-Beta | 37.0 | 95.0 | 3.0 | 2.0 |

| | | | | | |
|---|---|---|---|---|---|
| a-Mono-ether = 5-hydroxymethyl-2-butoxymethyl furan and Diether = 2,5-di-2-butoxymethyl-furan.- b-Di-Alcohol = 2,5-dihydroxymethyl-furan.- c- Others = Acetal + Dimer.- | | | | | |

### Example 10: This example illustrates the use of the materials of Examples 1 and 2 as catalysts ("CAT B") in the direct etherification of furfural with 1-butanol in a discontinuous or batch reactor.

In a two mouth glass reactor of 10 ml, one mouth connected to a condenser, and containing a magnetic bar, 140 mg of furfural, 3000 mg of 1-butanol and 50 mg of a catalyst are introduced as described in Examples 1 and 2 ("CAT B"). The second reactor mouth is closed by a septum system which allows taking samples at different time intervals. Next, the reaction temperature is raised to 100 °C, by immersing the reactor in a silicone bath with temperature control. The reaction mixture is stirred and samples are taken at various time intervals up to 7 hours of reaction. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:

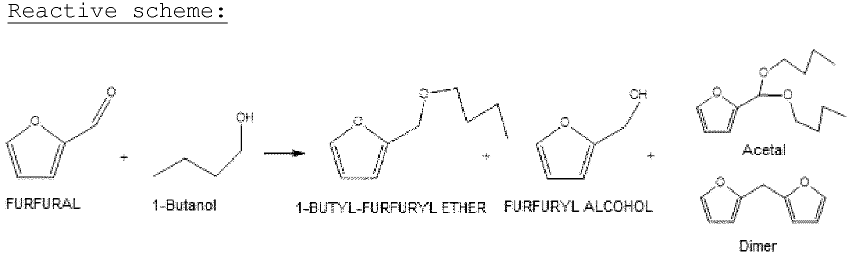

| **Catalyst** | | **Conversion (% Mol.)** | **Selectivity (% Mol.)** | | |
|---|---|---|---|---|---|
| **Example N°** | **Type** | | **Furfuryl ether^{a}** | **Acetal^{b}** | **Others^{c}** |
| 1 | Sn-Beta | 25.5 | 46.0 | 44.0 | 10.0 |
| 2 | Zr-Beta | 17.1 | 38.5 | 50.5 | 11.0 |

| | | | | | |
|---|---|---|---|---|---|
| a-Furfuryl ether = 1-butyl-furfuryl ether.- b- Acetal = 2-methyl-(1,1-dibutoxy)-furan. - c- Others = Furfuryl Alcohol + Dimer.- | | | | | |

### Example 11: This example illustrates the use of the materials of Examples 1 and 2, and combinations thereof, as catalyst ("CAT B") in the direct etherification of furfural with 2-butanol in a discontinuous or batch reactor.

In a two mouth glass reactor of 10 ml, one mouth connected to a condenser, and containing a magnetic bar, 180 mg of furfural, 1100 mg of 2-butanol and 100 mg of a catalyst or a catalyst mixture as those described in Examples 1 and 2 ("CAT B") are introduced. The second reactor mouth is closed by a septum system which allows taking samples at different time intervals. Next, the reaction temperature is raised to 100 °C, by immersing the reactor in a silicone bath with temperature control. The reaction mixture is stirred and samples are taken at various time intervals up to 7 hours of reaction. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:

| **Catalyst (physical mixture, mg)** | | **Conver sion (% Mol.)** | **Selectivity (% Mol.)** | | **Yield to furfuryl ether^{a} (% Mol.)** |
|---|---|---|---|---|---|
| **Sn-Beta (Ex. 1)** | **Zr-Beta (Ex. 2)** | | **Furfuryl ether^{a}** | **Furfuryl alcohol^{b}** | |
| 100 | 0 | 20 | 83 | 8 | 16.6 |
| 85 | 15 | 29 | 71 | 21 | 20.6 |
| 65 | 35 | 38 | 63 | 30 | 23.9 |
| 50 | 50 | 40 | 48 | 45 | 19.2 |
| 35 | 65 | 37 | 46 | 52 | 17.0 |
| 15 | 85 | 34 | 43 | 53 | 14.6 |
| 0 | 100 | 33 | 46 | 50 | 15.2 |

| | | | | | |
|---|---|---|---|---|---|
| a-Furfuryl ether = 2-butyl-furfuryl ether.- b- Furfuryl Alcohol = 2-hydroxymethylfuran.- | | | | | |

### Example 12: This example illustrates the use of the material prepared in Example 6 compared with the physical mixture 50:50 of the materials of Examples 1 and 2 as catalysts ("CAT B") in the direct etherification of furfural with 2-butanol in a discontinuous or batch reactor.

In a two mouth glass reactor of 10 ml, one mouth connected to a condenser, and containing a magnetic bar, 100 mg of furfural, 1100 mg of 2-butanol and 50 mg of a catalyst as the ones described in Examples 1, 2 and 6 ("CAT B"), or a 50:50 physical mixture of the materials of Examples 1 and 2, are introduced. The second reactor mouth is closed by a septum system which allows taking samples at different intervals. Next, the reaction temperature is raised to 100 °C, by immersing the reactor in a silicone bath with temperature control. The reaction mixture is stirred and samples are taken at various time intervals up to 7 hours of reaction. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:

| **Catalyst** | | **Conversion (% Mol.)** | **Selectivity (% Mol.)** | | |
|---|---|---|---|---|---|
| **Example N°** | **Type** | | **Furfuryl ether^{a}** | **Furfuryl alcohol^{b}** | **Others^{c}** |
| 1 | Sn-Beta | 27.2 | 70.0 | 22.0 | 8.0 |
| 2 | Zr-Beta | 48.2 | 63.0 | 30.0 | 7.0 |
| 6 | Sn-Zr-Beta | 26.2 | 56.0 | 37.0 | 8.0 |
| Sn-Beta + Zr-Beta (50:50) | | 33.6 | 87.4 | 10.0 | 2.6 |

| | | | | | |
|---|---|---|---|---|---|
| a-Furfuryl ether = 2-butyl-furfuryl ether.- b- Furfuryl alcohol = 2-hydroxymethylfuran.- c- Others = Acetal + Dimer.- | | | | | |

### Example 13: This example illustrates the use of the 50:50 physical mixture of the materials prepared in Examples 1 and 2 as a catalyst ("CAT B") in the direct etherification of furfural to 2-butanol, 2-hexanol and 2-octanol in a discontinuous or batch reactor.

In a two mouth glass reactor of 10 ml, one mouth connected to a condenser, and containing a magnetic bar, 180 mg of furfural, 1100 mg of 2-butanol, or 1520 mg of 2-hexanol or 1930 mg of 2-octanol, and 100 mg of a 50:50 physical mixture of the materials of Examples 1 and 2 ("CAT B") are introduced. The second reactor mouth is closed by a septum system which allows taking samples at different time intervals. Next, the reaction temperature is raised to 100 °C, by immersing the reactor in a silicone bath with temperature control. The reaction mixture is stirred and samples are taken at various time intervals up to 7 hours of reaction. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained: Catalyst: 50:50 physical mixture Sn-Beta (50 mg, Ex. 1) and Zr-Beta (50 mg, Ex. 2)

| **Solvent** | **Temperature (°C)** | **Conversion (% Mol.)** | **Selectivity (% Mol.)** | |
|---|---|---|---|---|
| | | | **Furfuryl ether^{a}** | **Furfuryl alcohol^{b}** |
| 2-butanol | 100 | 41,0 | 85,5 | 12,3 |
| 2-hexanol | 120 | 44,8 | 17,1 | 81,3 |
| | 130 | 41,8 | 11,0 | 84,8 |
| 2-octanol | 120 | 57,1 | 84,9 | 0 |
| | 130 | 80,7 | 89,7 | 0 |

| | | | | |
|---|---|---|---|---|
| a- Furfuryl ether = 2-alkyl-furfuryl ether.- b- Furfuryl Alcohol = 2-hydroxymethylfuran.- c- Others = Acetal + Dimer.- | | | | |

### Example 14: This example illustrates the use of the 50:50 physical mixture of the materials prepared in Examples 1 and 2 as a catalyst ("CAT B") in the direct etherification of furfural with 2-butanol in a fixed bed reactor and continuous feed.

In a tubular reactor of 20 cm stainless steel placed upright, fitted at the top with an inlet for continuously feeding liquid and/or gas, and with an outlet at the bottom, adapted to collect liquids with a refrigerated trap (at low temperature), 500 mg of a 50:50 physical blend of the materials of Examples 1 and 2 ("CAT B") previously tableted and sieved (mesh 0.4-0.6) are introduced. The catalyst is arranged in a bed combined with 500 mg pure Si carbide (0.2-0.4 mesh). The reactor is hermetically sealed, and then heated to 80 °C. The reaction liquid mixture (360 mg of furfural and 4400 mg 2-butanol) is controllably added via a perfusor syringe pump, with addition rates or flows comprised between 0.5 and 2.0 ml/h. Optionally, a carrier gas (N₂) is added at a flow of 3-4 ml/min by a mass controller. Liquid samples were collected at different time intervals until 7-8 reaction hours. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:
Catalyst: 50:50 physical mixture of Sn-Beta (250 mg, Ex. 1) and Zr-Beta (250 mg, Ex. 2)

| **Flow (ml/h)** | **W/F (g cat/mol Feeding/h )** | **Gas Carrier (ml/min)** | **Conversion (%Mol.)** | | **Selectivity to Fufuryl ether^{a} (% Mol.)** | |
|---|---|---|---|---|---|---|
| | | | **(0,66 hs)** | **(5 hs)** | **(0,66 hs)** | **(5 hs)** |
| 0.5 | 34.2 | --- | 99 | 86 | 83 | 62 |
| 0.5 | 34.2 | N₂ | --- | 100 | --- | 84 |
| 1.0 | 16.9 | --- | 100 | 80 | 83 | 38 |
| 1.0 | 16.9 | N₂ | 100 | 100 | 90 | 39 |
| 2.0 | 8.5 | --- | 79 | 62 | 53 | 18 |

| | | | | | | |
|---|---|---|---|---|---|---|
| a- Furfuryl ether = 2-butyl-furfuryl ether.- | | | | | | |

### Example 15: This example illustrates the use of the 50:50 physical mixture of the materials prepared in Examples 1 and 2 as a catalyst ("CAT B") in the direct etherification of furfuraldehyde with 2-butanol at various temperatures in a fixed bed reactor and continuous feed.

In a tubular reactor of 20 cm stainless steel placed upright, fitted at the top with an inlet for continuously feeding liquids and/or gas, and at the bottom with an outlet adapted to collect liquids with a refrigerated trap (at low temperature), 500 mg of a 50:50 physical blend of the materials of Examples 1 and 2 ("CAT B") previously tableted and sieved (mesh 0.4-0.6) are introduced. The catalyst is arranged in a bed combined with 500 mg pure Si carbide (0.2-0.4 mesh). The reactor is hermetically sealed, and then heated to the reaction temperature (60-80 °C). The liquid reaction mixture (360 mg furfural and 4400 mg of 2-butanol) is controllably added via a perfusor syringe pump at an addition rate or flow of 0.5 ml / h. Liquid samples at different time intervals are collected until 7-8 reaction hours. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:
Catalyst: 50:50 physical 50:50 Sn-Beta (250 mg, Ex. 1) and Zr-Beta (250 mg, Ex. 2)

| **Temperature (°C)** | **Conversion (%Mol.)** | | **Selectivity to Furfuryl ether^{a} (% Mol.)** | |
|---|---|---|---|---|
| | **(0.66 hs)** | **(5 hs)** | **(0.66 hs)** | **(5 hs)** |
| 80 | 99 | 86 | 83 | 62 |
| 70 | 100 | 97 | 71 | 47 |
| 60 | 100 | 97 | 31 | 29 |

| | | | | |
|---|---|---|---|---|
| a- Furfuryl ether = 2-butyl-furfuryl ether.- | | | | |

### Example 16: This example illustrates the use of the 50:50 physical mixture of the materials prepared in Examples 1 and 2 as a catalyst ("CAT B") in the direct etherification of furfural to 2-butanol, or 2-hexanol or 2-octanol in a fixed bed reactor and with continuous feeding.

In a tubular reactor of 20 cm stainless steel placed upright, fitted at the top with an inlet for continuously feeding liquid and / or gas, and at the bottom with an outlet adapted to collect liquids with a refrigerated trap (at low temperature), 500 mg of a 50:50 physical blend of the materials of Examples 1 and 2 ("CAT B") previously tableted and sieved (mesh 0.4-0.6) are introduced. The catalyst is arranged in a bed combined with 500 mg pure Si carbide (0.2-0.4 mesh). The reactor is hermetically sealed, and then heated to 80 °C. The liquid reaction mixture (360 mg furfural and 4400 mg of 2-butanol, or 6000 mg of 2-hexanol, or 7700 mg of 2-octanol) is controllably added via a perfusor syringe pump at an addition rate or flow of 0.5 ml / h. Liquid samples are collected at different time intervals until 7-8 reaction hours. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:
Catalyst: 50:50 physical mixture 50:50 Sn-Beta (250 mg, Ex. 1) and Zr-Beta (250 mg, Ex. 2)

| **Alcohol used** | **Conversion (%Mol.)** | | **Selectivity to furfuryl ether^{a} (% Mol.)** | |
|---|---|---|---|---|
| | **(0.66 hs)** | **(5 hs)** | **(0.66 hs)** | **(5 hs)** |
| 2-Butanol | 99 | 86 | 83 | 62 |
| 2-Hexanol | 86 | 65 | 92 | 84 |
| 2-Octanol | 100 | 100 | 92 | 91 |

| | | | | |
|---|---|---|---|---|
| a- Furfuryl ether = 2-alkyl-furfuryl ether.- | | | | |

### Example 17: This example illustrates the use of the 50:50 physical mixture 5of the materials prepared in Examples 1 and 2 as a catalyst ("CAT B") in the direct etherification of 2-octanol furfural in a fixed bed reactor and with continuous feeding for 200 hours.

In a tubular stainless steel reactor of 20 cm placed upright, fitted at the top with an inlet for continuously feeding liquid and/or gas, and at the bottom with an outlet adapted to collect liquids with a refrigerating trap (at low temperature), 500 mg of a 50:50 physical blend of the materials of Examples 1 and 2 ("CAT B") previously tableted and sieved (mesh 0.4-0.6) are introduced. The catalyst is arranged in a bed combined with 500 mg of pure Si carbide (0.2-0.4 mesh). The reactor is hermetically sealed, and then heated to 80 °C. The liquid reaction mixture (3900 mg of furfural and 79500 mg of 2-octanol) is controllably added via a perfusor syringe pump at an addition rate or flow of 0.5 ml/h. Liquid samples at different time intervals are collected up to 200 hours of reaction. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:
Catalyst: 50:50 physical mixture Sn-Beta (250 mg, Ex. 1) and Zr-Beta (250 mg, Ex. 2)

| **Time (hs.)** | **Conversion (% Mol.)** | **Selectivity to Furf. ether^{a} (%Mol.)** | **Time (hs.)** | **Conversion (% Mol.)** | **Selectivity to Furf. ether^{a} (%Mol.)** |
|---|---|---|---|---|---|
| 1 | 100 | 91 | 72 | 100 | 84 |
| 2 | 100 | 92 | 96 | 100 | 83 |
| 4 | 100 | 89 | 120 | 100 | 79 |
| 7 | 100 | 88 | 144 | 98 | 75 |
| 24 | 100 | 87 | 168 | 97 | 76 |
| 48 | 100 | 88 | 200 | 96 | 74 |

| | | | | | |
|---|---|---|---|---|---|
| a- Furfuryl ether = 2-octyl-furfuryl ether.- | | | | | |

### Example 18: This example illustrates the use of 5% Ru/C material as compared to other supported noble metal catalysts, as catalyst ("CAT A") in the reduction of 2-butyl-2-furfuryl ether with 2-butanol in the presence of H₂ and in a discontinous or batch reactor.

In a 3 ml glass reactor containing a magnetic stirrer, 1500 mg of a mixture containing 3.7% by weight of 2-butyl-furfuryl ether in 2-butanol, and 50 mg of metal catalyst ("CAT A ") were placed. The reactor is hermetically sealed, with the system containing a connection to a pressure gauge (manometer) system, another connection to load the gaseous source of hydrogen and a third outlet that allows taking samples at different time intervals. The reactor is pressurized to 5-15 bar with hydrogen and the reaction temperature is raised to 130 °C, by immersing the reactor in a silicone bath with temperature control. The reaction mixture is stirred and samples are taken at various time intervals up to 24 hours of reaction. The samples are analyzed using GC with an FID detector, calculating from the composition of the mixture obtained, the conversion of furan ether (initial moles of reactant - final moles of reactant / initial moles of reactant * 100), and the selectivities of the products obtained (moles of i product / moles of total products * 100) in each case. Thus the following results were obtained:

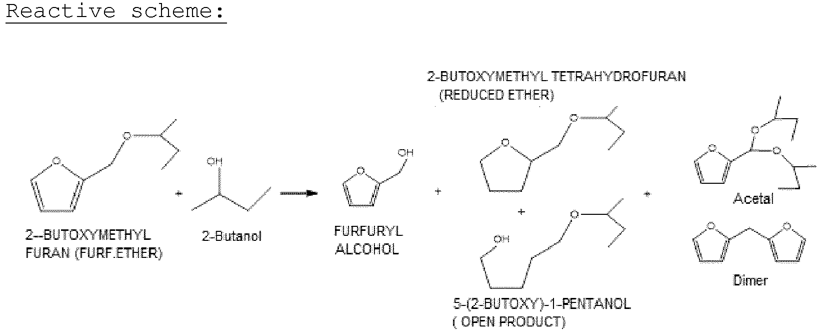

### Alcohol: 2-butanol

| **Catalyst** | **H₂ Pressure (bar)** | **Conversion (% Mol.)** | **Selectivity (% Mol.)** | | | |
|---|---|---|---|---|---|---|
| | | | **Furfuryl Alcohol^{a}** | **Ether Reduced^{b}** | **Prod. Open^{c}** | **Others^{d}** |
| 5%Ru/C | 5 | **82.9** | 6.2 | **71.4** | 4.9 | 17.5 |
| | 15 | **100.0** | 0 | **83.5** | 5.0 | 11.5 |
| 5%Pt/C | 5 | 36.4 | 50.5 | 31.0 | 4.7 | 13.8 |
| 5%Pt/Al₂O₃ | 5 | 46.6 | 46.7 | 27.7 | 3.3 | 22.3 |
| 2%Rh/Al₂O₃ | 15 | 9.6 | 57.6 | 19.3 | 9.5 | 13.6 |

| | | | | | | |
|---|---|---|---|---|---|---|
| a- Furfuryl alcohol = 2-hydroxymethylfuran.- b- Reduced ether = 2-(2-butoxy)-methyl-tetrahydrofuran.- c- Open product = 5-(2-butoxy)-1-pentanol.- d- Others = Acetal + Dimer.- | | | | | | |

### Example 19: This example illustrates the use of 5% Ru/C material as compared to other supported noble metal catalysts, as catalyst ("CAT A") in reduction of 2-octyl furfuryl ether with 2-octanol in the presence of H₂ and in a discontinuous or batch reactor.

In a 3 ml glass reactor containing a magnetic stirrer, 1500 mg a mixture containing 3.7 wt% of 2-octyl-furfuryl ether 2-octanol, and 50 mg of metal catalyst ("CAT A ") are introduced. The reactor is hermetically sealed, the system containing a connection to a pressure gauge (manometer), another connection to load the gaseous source of hydrogen and a third outlet that allows taking samples at different time intervals. The reactor is pressurized to 5-15 bar with hydrogen and the reaction temperature is raised to 130 °C, by immersing the reactor in a silicone bath with temperature control. The reaction mixture is stirred and samples are taken at various time intervals up to 24 hours of reaction. The samples are analyzed using GC with an FID detector, calculating from the composition of the mixture obtained, the conversion of furan ether (initial moles of reactant - final moles of reactant / initial moles of reactant * 100), and the selectivities of the products obtained (moles of i product / moles of total products * 100) in each case. Thus the following results were obtained:

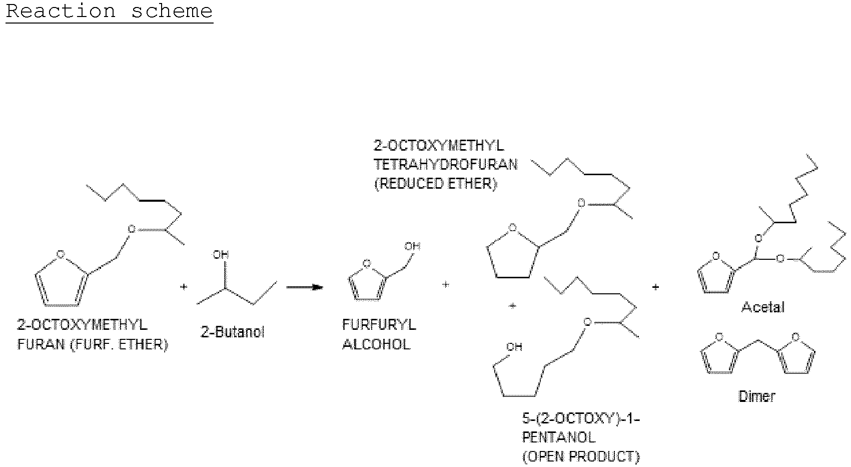

### Alcohol: 2-octanol

| **Catalyst** | **H₂ Pressure (bar)** | **Conversion (% Mol.)** | **Selectivity (% Mol.)** | | | |
|---|---|---|---|---|---|---|
| | | | **Furfuryl Alcohol^{a}** | **Reduced ether^{b}** | **Open Prod.^{c}** | **Others^{d}** |
| 5%Ru/C | 5 | **100.0** | 0 | **89.3** | 7.0 | 3.7 |
| | 15 | **100.0** | 0 | **88.4** | 10.4 | 1.2 |
| 5%Pt/C | 5 | 56.4 | 41.5 | 40.0 | 7.8 | 10.7 |

| | | | | | | |
|---|---|---|---|---|---|---|
| a- Furfuryl Alcohol = 2-hydroxymethylfuran.- b- Reduced ether = 2-(2-octoxy)-methyl-tetrahydrofuran.- c- Open Product = 5-(2-octoxy)-1-pentanol.- d- Others = Acetal + Dimer.- | | | | | | |

### Example 20: This example illustrates the use of one material prepared in Example 7 as a catalyst ("CAT C") in the reduction of butyl furfuryl-ether with 2-butanol in the presence of H₂ and in a discontinuous or batch reactor.

In a 3 ml glass reactor containing a magnetic stirrer, 1500 mg of a mixture containing 3.7% by weight of 2-butyl-furfuryl ether in 2-butanol, and 50 mg of the material prepared in Example 7 ("CAT C") were placed. The reactor is hermetically sealed, the system containing a connection to a pressure gauge (manometer), another connection to load the gaseous source of hydrogen and a third outlet that allows taking samples at different time intervals. The reactor is pressurized to 5 bar with hydrogen and the reaction temperature is raised to 130 °C, by immersing the reactor in a silicone bath with temperature control. The reaction mixture is stirred and samples are taken at various time intervals up to 24 hours of reaction. The samples are analyzed using GC with an FID detector, calculating from the composition of the mixture obtained, the conversion of furan ether (initial moles of reactant - final moles of reactant / initial moles of reactant * 100), and the selectivities of the products obtained (moles of i product / moles of total products * 100) in each case. Thus the following results were obtained:

### Alcohol: 2-butanol

| **Catalyst** | **Conversion (% Mol.)** | **Selectivity (% Mol.)** | | | |
|---|---|---|---|---|---|
| | | **Furfuryl alcohol^{a}** | **Reduced ether^{b}** | **Open Prod. ^{c}** | **Others^{d}** |
| 1,4%Pt/Sn-Beta | 26.3 | 20.9 | 46.6 | 5.5 | 27.0 |

| | | | | | |
|---|---|---|---|---|---|
| a- Furfuryl Alcohol = 2-hydroxymethylfuran.- b- Reduced ether = 2-(2-butoxy)-methyl-tetrahydrofuran.- c- Open product = 5-(2-butoxy)-1-pentanol.- d- Others = Acetal + Dimer.- | | | | | |

### Example 21: This example illustrates the use of 5% Ru/C (CAT A) material and the 50:50 physical mixture of materials prepared in Examples 1 and 2 (CAT B) as catalyst ("CAT A" + "CAT B ") in the etherification / reduction cascade of furfural with 2-butanol in the presence of H₂ and in a discontinuous or batch reactor.

In a 3 ml glass reactor containing a magnetic stirrer, 100 mg of furfural, 1100 mg of 2-butanol, 50 mg of a 50:50 physical blend of the materials of Examples 1 and 2 ("CAT B "), and 25 mg of a catalyst as described in Example 7 (" CAT C "), or 25 mg of a catalyst of 5% Ru/C (" CAT A ") type, were placed. The reactor is hermetically sealed, the system containing a connection to a pressure gauge (manometer), another connection to load the gaseous source of hydrogen and a third outlet that allows taking samples at different time intervals. The reactor is heated to 100 °C by immersing the same in a silicone bath with temperature control. The reaction mixture is stirred and samples are taken at various time intervals up to 24 hours of reaction. After 24 hours, the reactor is pressurized at 5-15 bar with hydrogen and the reaction temperature is raised to the 100-140 °C. The reaction mixture is stirred and samples taken at various time intervals for additional 24 reaction hours. The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:

### Alcohol: 2-butanol

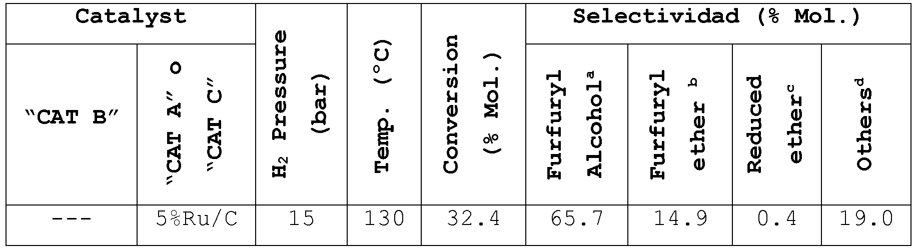

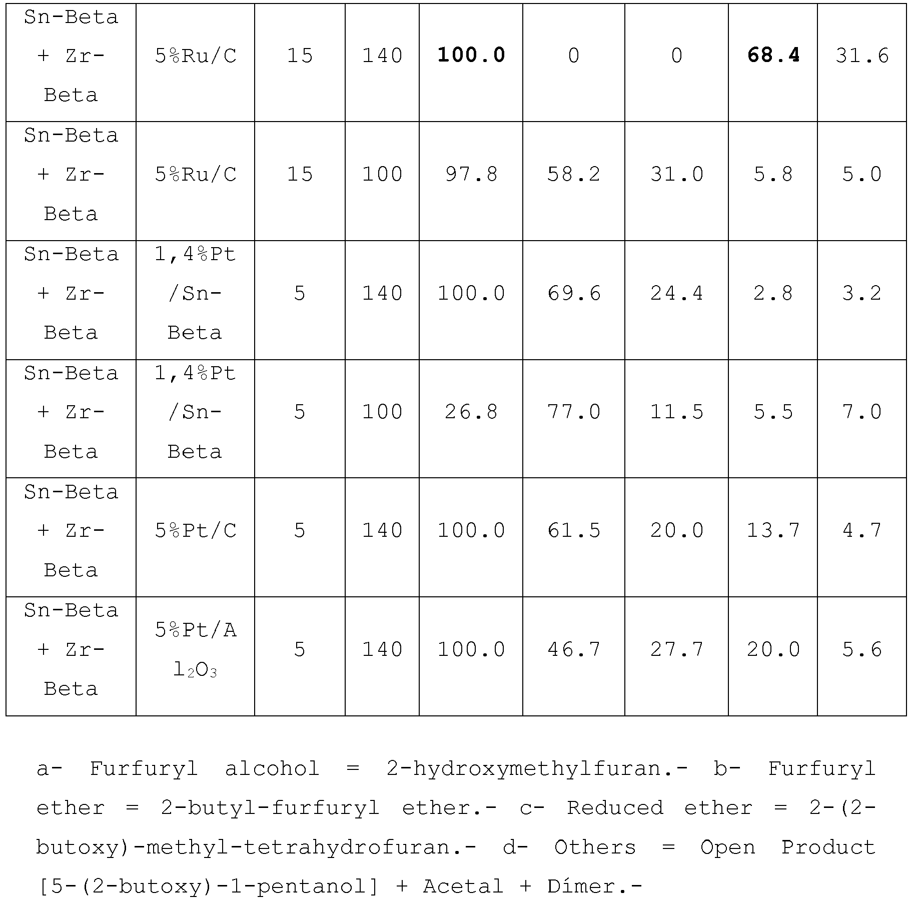

### Example 22: This example illustrates the use of the 50:50 mixture of the materials prepared in Examples 1 and 2 (CAT B) and the 5% Ru/C (CAT A) material as catalysts in consecutive etherification reactions (Reaction 1, batch reactor) and subsequent reduction (Reaction 2, batch or discontinuous reactor) of furfural to 2-butanol and 2-octanol

In a stainless steel autoclave reactor 15 ml, inside covered with Teflon and containing a magnetic stirrer, 460 mg of furfural, 5500 mg of 2-butanol or 9650 mg of 2-octanol, and 250 mg of a 50:50 physical mixture of materials of Examples 1 and 2 ("CAT B"), were placed. The reactor is hermetically sealed, the system containing a connection to a pressure gauge (manometer), another connection for charging gases and a third outlet that allows taking samples at different time intervals. The reactor is heated to 100 °C by immersing the same in a silicone bath with temperature control. The reaction mixture is stirred and samples taken at various time intervals up to 24 hours of reaction (Reaction 1). After 24 hours, the reactor is opened, the reaction mixture is filtered and put back into the reactor, and 125 mg of a catalyst of 5% Ru/C type ("CAT A") are added. The reactor is hermetically sealed and pressurized to 5 bar with hydrogen and the reaction temperature is raised to 130 °C. The reaction mixture is stirred and samples were taken at various time intervals for additional 24 hours reaction (Reaction 2). The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus, the following results were obtained:

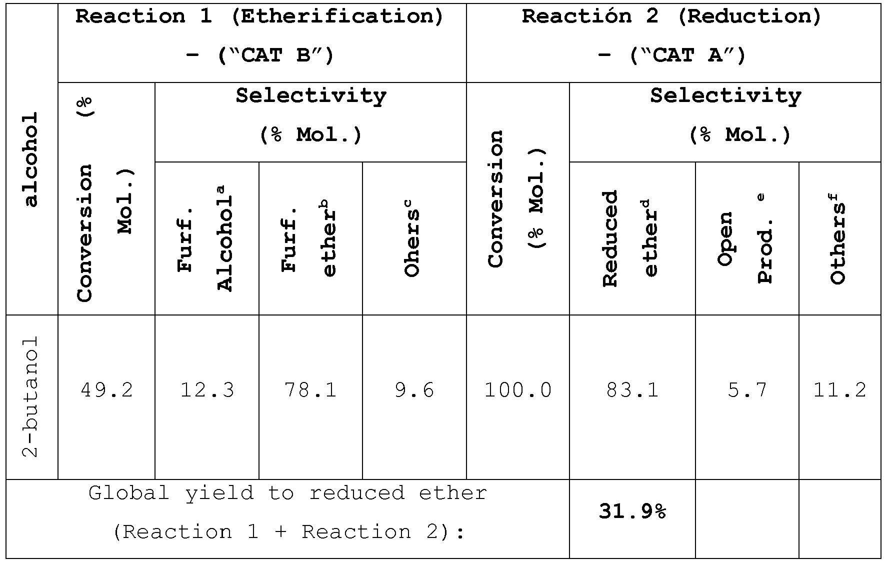

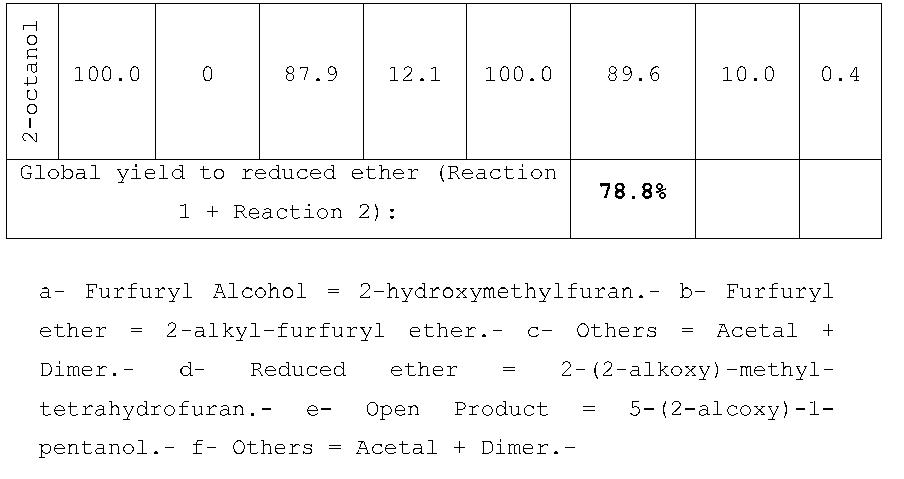

### Example 23: This example illustrates the use of the 50:50 mixture of the materials prepared in Examples 1 and 2 (CAT B) and of the 5%Ru/C (CAT A) as catalysts in consecutive etherification (Reaction 1 fixed-bed reactor with continuous feeding) and subsequent reduction reaction (Reaction 2, batch reactor) of furfural with 2-butanol.

In a stainless steel tubular reactor of 20 cm, placed upright, fitted at the top with an inlet for continuously feeding liquid and/or gas, and at the bottom with an outlet adapted to collect liquids with a refrigerating trap with (at low temperature), 750 mg of a 50:50 physical blend of the materials of Examples 1 and 2 ("CAT B") previously tableted and sieved (mesh 0.4-0.6) are introduced. The catalyst is arranged in a bed combined with 750 mg of pure Si carbide (0.2-0.4 mesh). The reactor is hermetically sealed, and then heated to 80 °C. The liquid reaction mixture (3900 mg 79500 mg of furfural and 2-octanol) is controllably added via a perfusor syringe pump at an addition rate or flow of 0.62 ml/h. Liquid samples at different time intervals are collected until 200 hours of reaction, up tp a total of 82200 mg, divided into 3 parts of 27400 mg (Reaction 1). Each of these aliquots were introduced into a reinforced glass reactor of 100 ml, containing a magnetic stirrer, along with 500 mg of a catalyst of 5% Ru/C type ("CAT A"). The reactor is hermeticaly sealed, the lid containing a connection to a pressure gauge (manometer), another connection to load the hydrogen source and a third outlet that allows taking samples at different time intervals. The reactor is pressurized to 5 bar with hydrogen and heated to 130 °C by immersing the same in a silicone bath with temperature control. The reaction mixture is stirred and samples are taken at various time intervals up to 24 hours of reaction.
(Reaction 2). The samples are analyzed using GC with an FID detector, calculating from the composition of the mixture obtained, the conversion of furan compound (initial moles of reactant - final moles of reactant / initial moles of reactant * 100), and the selectivities of the products obtained (moles of i product / moles of total products * 100) in each case. Thus the following results were obtained:

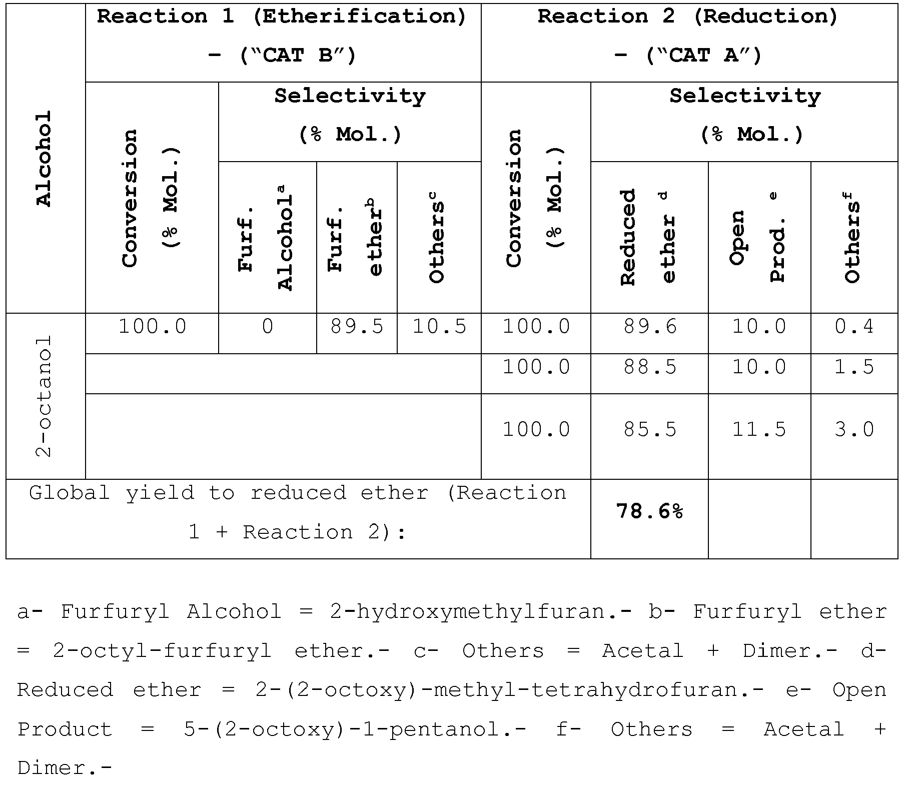

## Claims

1. Process for the preparation of tetrahydro-furfuryl ethers, comprising performing consecutive etherification / reduction reactions in cascade, of at least one compound containing one or more furan rings in the absence of solvent, and in the presence of at least:
- one or more alcohols,
- one or more catalysts,
where the etherification reaction is carried out in the absence of hydrogen and the reduction reaction is carried out in the presence of hydrogen.

2. Process according to claim 1, wherein a compound of tetrahydro-furfuryl ether type, containing at least one tetrahydrofuran ring, and having the general formula: 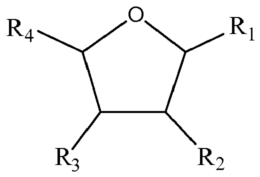 is obtained, wherein R₁ is a (-H₂C-OR group, R being an alkyl of 1 to 24 carbon atoms, linear or branched, substituted or non-substituted; cyclic alkyl having 4 to 24 C atoms, substituted or non-substituted; or aryl having 6 to 18 carbon atoms, substituted or non-substituted) ; and R₂, R₃ and R₄ are the same or different substituents and are selected from among hydrogen, alcohol (H₂C-OH), alkoxy, (H₂C-OR, R being an alkyl or aryl group), carboxyl (-COOH), carboxylate (COOR, where R is an alkyl or aryl group), aliphatic or aromatic with 4 to 12 carbon atoms oxygenated heterocycle, substituted or unsubstituted, alkyl having 1 to 24 carbon atoms, linear or branched , substituted or unsubstituted; cyclic alkyl with 4 to 24 C atoms, substituted or unsubstituted; or aryl with 6 to 18 C atoms, substituted or unsubstituted.

3. Process according to claim 2, wherein a compound of tetrahydro-furfuryl ether type having 6 to 24 carbon atoms is obtained.

4. Process according to claim 2 wherein the furan compound contains a furan ring (mono-furan).

5. Process according to claim 4, wherein the mono-furan compound is selected from furfural, 5-hydroxymethylfurfural, 5-methoxymethyl furfural, 5-ethoxymethyl furfural, and combinations thereof.

6. Process according to claim 1 wherein the alcohol used corresponds to the formula: 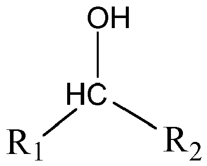 wherein R₁ and R₂ are the same or different from each other, and are selected from hydrogen, alkyl having 1 to 24 carbon atoms, linear or branched, substituted or unsubstituted; cyclic alkyl with 4 to 24 C atoms, substituted or unsubstituted; or aryl with 6 to 18 C atoms, substituted or unsubstituted.

7. Process according to claim 6, wherein the alcohol is selected from: an aliphatic primary alcohol having 2 to 12 carbon atoms, an aliphatic secondary alcohol having 2 to 12 carbon atoms, and combinations thereof.

8. Process according to claim 1, wherein the hydrogen is derived from a source selected from molecular hydrogen, a gas mixture containing hydrogen, and combinations thereof.

9. Process according to claim 1 wherein the catalyst is selected from:
a) a metallic catalyst "CAT A" comprising one or more noble metals, or one or more transition metals, or one or more of its salts or complexes, and combinations thereof, with the aforementioned "CAT A" being supported, or included in a carbonaceous type solid or in the structure of an inorganic matrix;
b) a metallic catalyst "CAT B" comprising one or more transition metals, their salts or complexes, included within or supported on an inorganic matrix structure;
c) a metallic catalyst "CAT C" comprising at least one noble metal and one or more transition metals, or one or more of its salts or complexes, and combinations thereof, said "CAT C" being supported, or included in the structure of an inorganic matrix;
d) combinations thereof.

10. Procees according to claim 9, wherein the noble metal in the catalyst "CAT A" is selected from Au, Pd, Ag, Pt, Ru, Re, Rh, or combinations thereof.

11. Process according to claim 10, wherein said metal is Ru or Ru combined with another metal.

12. Process according to claim 9 wherein the transition metal in the catalyst "CAT A" is selected from Ti, Zr, Zn, Cu, Co, Mn, Mo, V, Ni, Fe, Al, and combinations thereof.

13. Process according to claim 9 wherein the transition metal in the catalyst "CAT B" and catalyst "CAT C" is selected from Si and Sn, Zr, Ti, Ga, Ta, Al, or combinations thereof.

14. Process according to claim 9, wherein the noble metal in the catalyst "CAT C" is selected from Au, Pd, Ag, Pt, Ru, Re, Rh, or combinations thereof.

15. Process according to claim 14, wherein the noble metal is Pt or Pd combined with a second metal.

16. Process according to claim 9, wherein said carbonaceous solid has a surface area between 50 and 1200 m²/g and is a material selected from: carbon, active carbons, carbon nanotubes, graphene, carbon nitrides, and combinations thereof.

17. Process according to claim 9 wherein the inorganic matrix is an amorphous material selected from one or more metal oxides, one or more mixed metal oxides, and combinations thereof.

18. Process according to claim 17, wherein said inorganic matrix is selected from: silica, alumina, ceria, yttria, titania, Fe₂O₃, silica-alumina, silica-ceria, one or more mixed oxides of alkaline earth metals, one or more transition metal oxides.

19. Process according to claim 17, wherein said inorganic matrix is an amorphous siliceous material comprising Si and an element selected from Sn, Zr, Ti, Ga, Ta, Al, or combinations thereof.

20. Process according to claim 9 wherein said inorganic matrix is one or more microporous molecular sieves.

21. Process according to claim 20, wherein the microporous molecular sieve has, in its calcined and anhydrous state, the following chemical composition:
y(A_{1/n}ⁿ⁺ XO₂) : t TO₂ : SiO₂: x SnO₂
in which,
- X represents at least one trivalent element, preferably selected from Al, or Ga, or Ta, or combinations thereof,
- "y" is a number comprised between 0 and 0.2,
- A represents a mono-, di-, or trivalent cation, or combinations thereof,
- n = 1, 2 or 3,
- T represents at least one tetravalent element other than Si and Sn, preferably selected from Ti or Zr, or combinations thereof,
- "t" is a number comprised between 0 and 0.2,
- "x" is a number comprised between 0 and 0.2, and preferably between 0.001 and 0.1.

22. Process according to claims 20 and 21, wherein said microporous molecular sieve has a structre corresponding to a zeolite selected from a Beta zeolite, Mordenite and ITQ-16.

23. Process according to one of claims 20 to 22, **characterized in that** said Beta zeolite the Si atoms are partially replaced by Sn, or Zr, Ti, Ga, or Ta, or Al, or combinations thereof.

24. Process according to claim 9 wherein said inorganic matrix is one or more mesoporous molecular sieves.

25. Process according to claim 24, wherein said mesoporous molecular sieve is selected from among silicate, metal-silicate and a meso-porous material derived from the delamination of a laminar precursor.

26. Process according to one of claims 24 and 25 wherein the mesoporous molecular sieve has, in its calcined and anhydrous state, the following chemical composition:
y(Aⁿ⁺_{1/n} XO₂) : t TO₂ : (1-m) S, O₂: x SnO₂ : m R₍₄₋ₚ₎ SiO_{P/2} : s S
in which
- A represents one or more of mono-, di- or trivalent compensating cations, or combinations thereof,
- X represents at least one trivalent element, preferably selected from Al, Ga, or Ta, or combinations thereof
- "y" is a number comprised between 0 and 0.2,
- n = 1, 2 or 3,
- T represents at least one tetravalent element other than Si and Sn, preferably selected from Ti or Zr, or combinations thereof,
- "t" is a number comprised between 0 and 1, and preferably between 0 and 0.2,
- "x" is comprised between 0 and 0.2,
- S represents an organic compound,
- "s" is a number ranging between 0 and 0.5.
- "m" is a number comprised between 10-6 and 0.66,
- "p" is a number comprised between 3 and 1,
- and where R is an alkyl, aromatic group or a combination thereof derived from the silylation agent containing the Si-C bonds.

27. Process according to one of claims 24 to 26, wherein said mesoporous molecular sieve is selected from materials of type of MCM-41, MCM-48, SBA-15, HMS and mixtures thereof.

28. Process according to one of claims 24 to 27, wherein in said mesoporous molecular sieve the Si atoms are partially replaced by Sn, or Zr, Ti, Ga, or Ta, or Al, or combinations thereof.

29. Process according to claim 1 wherein the etherification / reduction reactions of furan compounds are carried out consecutively in cascade ("one-pot") in a reactor selected from a batch reactor, a continuous stirred tank reactor (CSTR), a fixed-bed continuous reactor, fluidized bed reactor and boiling bed reactor.

30. Process according to claim 1, wherein the consecutive etherification / reduction reactions of furan compounds are carried out with a weight ratio of the furan compound to catalyst between 1 and 200.

31. Process according to claim 1, wherein the consecutive etherification / reduction reactions of furan compounds are carried out with a weight ratio of the alcohol to furan compound between 2 and 200.

32. Process according to claim 1, wherein the consecutive etherification / reduction reactions of furan compounds are carried out at a temperature comprised between 20 and 250 °C.

33. Process according to claim 1 wherein the consecutive etherification / reduction reactions of furan compounds are carried out in a reaction time comprised between 2 minutes and 200 hours.

34. Process according to claim 1 wherein the consecutive etherification / reduction reactions of furan compounds are carried out at a total pressure in the system between atmospheric pressure and 50 bar.
